(19) Europäisches Patentamt
European Patent Office
Office européen des brevets

(11) EP 1 664 079 B1

(12) **EUROPEAN PATENT SPECIFICATION**

(45) Date of publication and mention of the grant of the patent:
**30.04.2008 Bulletin 2008/18**

(21) Application number: **04763197.3**

(22) Date of filing: **13.07.2004**

(51) Int Cl.:
*C07J 43/00* (2006.01) *A61K 31/58* (2006.01)
*A61P 15/08* (2006.01)

(86) International application number:
**PCT/EP2004/007737**

(87) International publication number:
**WO 2005/014613 (17.02.2005 Gazette 2005/07)**

(54) **THIOMORPHOLINO STEROID COMPOUNDS, THE USE THEREOF FOR THE PREPARATION OF MEIOSIS-REGULATING MEDICAMENTS AND METHOD FOR THE PREPARATION THEREOF**

THIOMORPHOLINOSTEROIDVERBINDUNGEN, DEREN VERWENDUNG ZUR HERSTELLUNG VON DIE MEIOSE STEUERNDEN MEDIKAMENTEN UND VERFAHREN ZU DEREN HERSTELLUNG

COMPOSES THIOMORPHOLINO STEROIDES, LEUR UTILISATION POUR LA PREPARATION DE MEDICAMENTS REGULANT LA MEIOSE ET PROCEDE DE PREPARATION ASSOCIE

(84) Designated Contracting States:
**AT BE BG CH CY CZ DE DK EE ES FI FR GB GR HU IE IT LI LU MC NL PL PT RO SE SI SK TR**

(30) Priority: **28.07.2003 EP 03090237**

(43) Date of publication of application:
**07.06.2006 Bulletin 2006/23**

(73) Proprietor: **Bayer Schering Pharma Aktiengesellschaft**
**13353 Berlin (DE)**

(72) Inventors:
- **BLUME, Thorsten**
  **16552 Schildow (DE)**
- **LINDENTHAL, Bernhard**
  **13507 Berlin (DE)**
- **PRELLE, Katja**
  **13465 Berlin (DE)**
- **PETERS-KOTTIG, Michaele**
  **10115 Berlin (DE)**

(56) References cited:
**EP-A- 1 245 572** **WO-A-99/45024**
**DE-A- 1 930 473** **US-A- 2 813 094**
**US-A- 2 846 432**

- **BIVENS ET AL: 'A synthetic analog of meiosis-activating sterol (FF-MAS) is a potent agonist promoting meiotic maturation and preimplantation development of mouse oocytes maturing in vitro' HUMAN REPRODUCTION vol. 19, no. 10, 27 July 2004, pages 2340 - 2344**

Remarks:
The file contains technical information submitted after the application was filed and not included in this specification

EP 1 664 079 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

The invention relates to pharmaceutically active thiomorpholino steroid compounds, pharmaceutical compositions comprising these compounds, the use of these compounds for the preparation of a pharmaceutical composition being suitable to regulate reproduction, especially meiosis, of a contraceptive or as a profertility drug, a method for regulating reproduction, e.g. meiosis, a method for improving the possiblity of an oocyte's ability to develop into a mammal using these compounds as well as a method for the preparation of (20S)-20-[(thiomorpholin-4-yl)methyl]-4,4-dimethyl-5α-pregna-8,14-dien-3β-ol.

Meiosis is the unique and ultimate event of germ cells, on which sexual reproduction is based. Meiosis comprises two meiotic divisions. During the first division, exchange between maternal and paternal genes takes place, before the pairs of chromosomes are separated into the daughter cells. These contain only half the number (1n) of chromosomes and 2c DNA. The second meiotic division proceeds without a DNA synthesis. This division therefore results in the formation of the haploid germ cells with only 1c DNA.

The meiotic events are similar in the male and female germ cells, but the time schedule and the differentiation processes, which lead to ova and to spermatozoa differ profoundly. All female germ cells enter the prophase of the first meiotic division early in life, often before birth, but all are arrested as oocytes later in the prophase (dictyate state) until ovulation after puberty. Thus, from early life the female has a stock of oocytes, which is drawn upon until the stock is exhausted. Meiosis in females is not completed until after fertilization, and results in only one ovum and two abortive polar bodies per germ cell. In contrast, only some of the male germ cells enter meiosis from puberty and leave population of germ cells throughout life. Once initiated, meiosis in the male cell proceeds without significant delay and produces four spermatozoa.

Only little is known about the mechanisms, which control the initiation of meiosis in the male and in the female. New studies indicate that follicular purines, hypoxanthine and adenosine could be responsible for meiotic arrest in the oocyte [S.M. Downs et al., Dev. Biol., 82, 454-458 (1985); J.J. Epplg. et al., Dev. Biol., 119, 313-321 (1986); S.M. Downs, Mol. Reprod. Dev., 35, 82-94 (1993)]. The presence of a diffusible meiosis regulating substance was first described by Byskov *et al.* in a culture system of fetal mouse gonads [A.G. Byskov et al., Dev. Biol., 52, 193-200 (1976)]. A meiosis activating substance (MAS) is secreted by the fetal mouse ovary, in which meiosis is ongoing, and a meiosis preventing substance (MPS) is released from the morphologically differentiated testis with resting, non-meiotic germ cells. It was suggested that the relative concentrations of MAS and MPS regulate the beginning, arrest and resumption of meiosis in the male and in the female germ cells [A.G. Byskov et al, in: The Physiology of Reproduction (eds. E. Knobil and J.D. Neill), Raven Press, New York (1994)]. Clearly, if meiosis can be regulated, reproduction can be controlled. In a recent article [A.G. Byskov et al., Nature, 374, 559-562 (1995)] the isolation of certain sterols is described. Such sterols were isolated from bull testes and from follicular fluid and activate oocyte meiosis [T-MAS (testes meiosis-activating sterol) and FF-MAS (follicular fluid meiosis-activating sterol): 4,4-dimethyl-5α-cholesta-8,14,24-trien-3β-ol].

It was also demonstrated that micromolar concentrations of synthetic FF-MAS are able to induce resumption of meiosis in a dose-dependent manner in rat oocytes that are arrested by the phosphodiesterase inhibitor IBMX (3-isobutyl-1-methyl xanthine) [C. Hegele-Hartung et al., Biol. Reprod., 64, 418-424 (2001)]. It was shown that this effect can be observed when CEO (cumulus-enclosed oocytes) and DO (denuded oocytes) are cultured *in vitro* in the presence of FF-MAS.

Further substances that regulate the meiosis are descibed in the following documents.

In WO 98/52965 A1 meiosis activating 20-aralkyl-5α-pregnane derivatives are described.

In WO 00/68245 A1 steroid compounds are disclosed, which are able to inhibit meiosis such that these compounds are useful as contraceptives in females and males. These compounds are primarily unsaturated cholestan derivatives characterized by a 3β-hydrogen atom bonded to the $C^{14}$ carbon atom of the cholestan skeleton.

In WO 96/00235 A1 meiosis inducing sterols, being known as intermediates in the biosynthesis of cholesterol, as well as certain structurally related synthetic sterols, are described. These substances have been found to regulate meiosis. Similar to cholesterol these sterols are provided with a side chain on $C^{17}$ in the sterol skeleton and further with at least one of a $\Delta^7$, $\Delta^8$ or $\Delta^{8(14)}$ double bond.

In WO 96/27658 A1 a method of stimulating meiosis of a germ cell is disclosed, which comprises administering to the cell *in vivo,* ex *vivo* or *in vitro* an effective amount of a compound, which causes accumulation of an endogenous meiosis activating substance to a level, at which meiosis is induced. Such compounds which cause accumulation of the meiosis activating substance are disclosed to be amphotericin B, aminoguanidine, 3β,5α,6β-trihydroxycholestane, melatonine, 6-chloromelatonine and 5-methoxytryptamine as well as other derivatives and agonists thereof. Meiosis activating substances are also reported to be *inter alia* 5α-cholestan-3β-ol, D-homo-cholesta-8,14-dien-3β-ol and 22,25-diazacholestrol, 25-aza-24,25-dihydrolanosterol, 24,25-Iminolanosterol, 23- and 24-azacholestrol as well as 25-azacholestanol derivatives.

In WO 97/00884 A1 and in WO 98/28323 A1 substances are described which can be used for stimulating meiosis *in vitro, in vivo* or ex *vivo.* The compounds disclosed are hence agonists of naturally occurring meiosis activating

substances and may therefore be used in the treatment of infertility which is due to insufficient stimulation of meiosis in females and males. In this document also some compounds are disclosed which may be antagonists of naturally occurring meiosis activating substances, such that these compounds may be suitable for use as contraceptives. The compounds disclosed *inter alia* comprise 5α-cholest-8-ene-3β-ols and 5α-cholest-8,14-dien-3β-ols which *inter alia* may be provided with an amino group in the side chain bonded to $C^{17}$ of the cholesterol skeleton, the amino group being bonded to the sterol skeleton via a $C_4$-spacer. $C_1$ - $C_4$ alkyl or $C_3$ - $C_6$ cycloalkyl are bonded to the amino group.

Further in WO 99/58549 A1 sterol derivatives are disclosed which are effective in regulating meiosis. These compounds are described to have the ability to relieve infertility in females and males, particularly in humans. The sterol derivatives being effective as regulating substances are *inter alia* (20R)-20-methyl-23-dimethylamino-5a-pregna-8,14-dien-3β-ol, (20R)-20-methyl-23-dimeihylamino-5α-pregna-5,7-dien-3β-ol, 4,4-dimethyl-24-phenylamino-5α-chola-8,14-dien-3β-ol, 4,4-dimethyl-24-(N,N-dimethylamino)-24-cyano-5α-cholesta-8,14-dien-3β-ol and further a variety of 24-oic acid amides of sterols having one or more double bonds in the sterol skeleton.

In WO 02/079220 A2 steroid compounds are described which have meiosis regulating ability, these compounds being primarily sterols having an aminomethyl or aminoethyl moiety bound to $C^{20}$. The amino group may be *e.g.* a nitrogen containing heterocyclic ring, more specifically a piperidin ring. One example of such compounds is (20S)-20-[(piperidin-1-yl)methyl]-4.4-dimethyl-5α-pregna-8,14-dien-3β-ol. In general these compounds exhibit a meiosis stimulating effect in oocytes, especially in CEOs (= cumulus enclosed oocytes). In one specific example a thiomorpholino compound is disclosed.

Unsaturated sterol derivatives having an amino group in the side chain at $C^{17}$ have also been described by: J.J. Sheets and L.E. Vickery in: "Active Site-directed Inhibitors of Cytochrome P-450scc" in J. Biol. Chem., Vol.258 (19), 1983, pages 11446 -11452 with regard to the effect of these sterols on bovine adenocortical cholesterol side chain cleavage cytochrome P-450 (P-450 scc). In this document *inter alia* 22-amino-23,24-bis-nor-chol-5-en-3β-ol and 23-amino-24-norchol-5-en-3β-ol are disclosed.

Further unsaturated derivatives having an amino group in the side chain at $C^{17}$ have been reported by: A.T. Mangla and W.D. Nes in: "Sterol C-methyl Transferase from Prototheca wickerhamii, Mechanism, Sterol Specificity and Inhibition" in Bioorg. and Med. Chem. (2000), 8 (5), 925 - 936. In this document *inter alia* 23-aza-zymosterol is disclosed.

It has been discovered, when using previously described meiosis regulating components that resumption of meiosis occurs in naked oocytes *in vitro.* However, many of these compounds were only marginal effective when stimulating meiosis in oocytes surrounded by granulosa cells (CEOs). Further rates of *in vitro* fertilization and of retransfer implantations and also the number of fetuses alive at the end of pregnancy is not sufficiently high.

One object of the present invention is to find substances that are useful for regulating reproduction, in particular meiosis, in females and males, especially in mammals and more specifically in humans.

It is another object of the present invention to provide a novel pharmaceutical composition comprising the novel substances.

It is another preferred object of the present invention to provide a use of the novel substances for the preparation of a pharmaceutical composition that is suitable to regulate reproduction, especially meiosis.

It is a further object of the present invention to improve maturation of human oocytes.

It is still another object of the present invention to improve synchrony of nuclear, cytoplasmic and/or membranous oocyte maturation.

It is still another object of the present invention to improve fertility of oocytes.

It is still another object of the present invention to improve the rate of implantation of oocytes by human *in vitro* maturation and fertilization.

It is still a further object of the present invention to diminish the incidence of human pre-embryos with chromosome abnormalities (aneuploidy).

It is still a further object of the present invention to improve the cleavage rate of human pre-embryos.

It is still a further object of the present invention to improve the quality of human pre-embryos.

It is another object of the present invention to provide a method for the preparation of the novel substances.

According to the present invention thiomorpholine steroid compounds of general formula I may advantageously be employed in regulating the reproduction, *e.g.* meiosis, in mammals, *e.g.* in females and males, and in particular in humans:

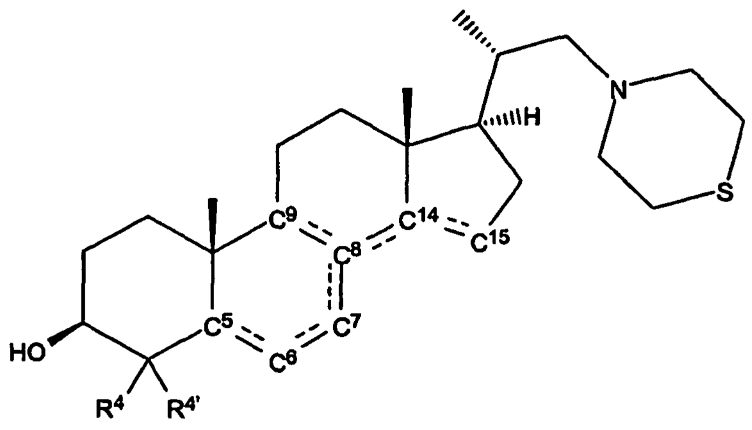

I

wherein in the moiety **I'** of compound **I**

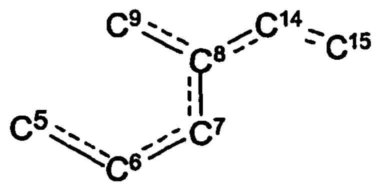

I'

each bond between between $C^5$ and $C^6$, between $C^6$ and $C^7$, between $C^7$ and $C^8$, between $C^8$ and $C^9$, between $C^8$ and $C^{14}$ and between $C^{14}$ and $C^{15}$, independently, is a single bond or a double bond, at least one of these bonds being a double bond, and wherein each carbon atom $C^5$, $C^6$, $C^7$, $C^8$, $C^9$, $C^{14}$ and $C^{15}$ is bonded to each neighbouring C atom at the most by one double bond, with the proviso that there is no double bond in the steroid skeleton exclusively between $C^5$ and $C^6$ (which latter condition means that compounds exclusively having a $\Delta^5$ double bond are not comprised by the present invention), and

wherein

$R^4$ and $R^{4'}$, independently, are selected from the group comprising hydrogen and methyl.

The moiety with general formula **I'** in the steroid compound according to the present invention preferably comprises one double bond between $C^8$ and $C^{14}$ or two conjugated double bonds, preferably either two double bonds between $C^8$ and $C^9$ and between $C^{14}$ and $C^{15}$ or two double bonds between $C^5$ and $C^6$ and between $C^7$ and $C^8$.

$R^4$ and $R^{4'}$ are preferably the same radicals, *i.e.* they are both hydrogen or both methyl.

For the rest hydrogen atoms may be bonded to the $C^1$, $C^2$, $C^5$, $C^6$, $C^7$, optionally $C^8$, $C^9$, $C^{11}$, $C^{12}$, $C^{14}$, $C^{15}$, $C^{16}$ and $C^{17}$, depending on whether the respective C atoms are part of a double bond or not. In $C^{10}$, $C^{13}$ and $C^{18}$ methyl groups are bonded to the steroid skeleton and the side chain, respectively.

More preferably according to the present invention the steroid compound is selected from the group comprising:

(20S)-20-[(thiomorpholin-4-yl)methyl]-4,4-dimethyl-5α-pregna-8,14-dien-3β-ol:

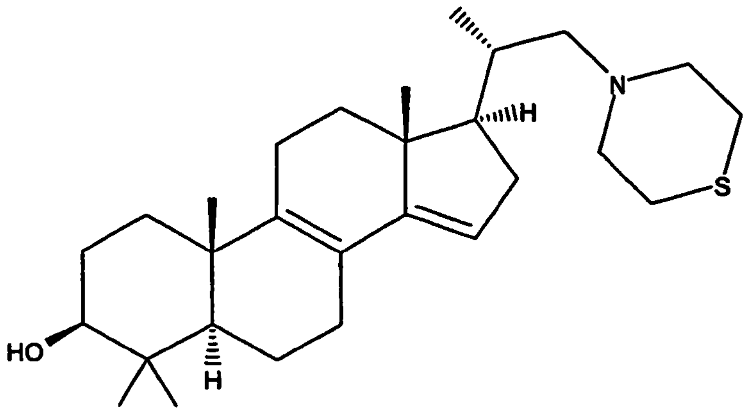

**Compound IA**

(20S)-20-[(thiomorpholin-4-yl)methyl]-4,4-dimethyl-5α-pregna-8(14)-en-3β-ol:

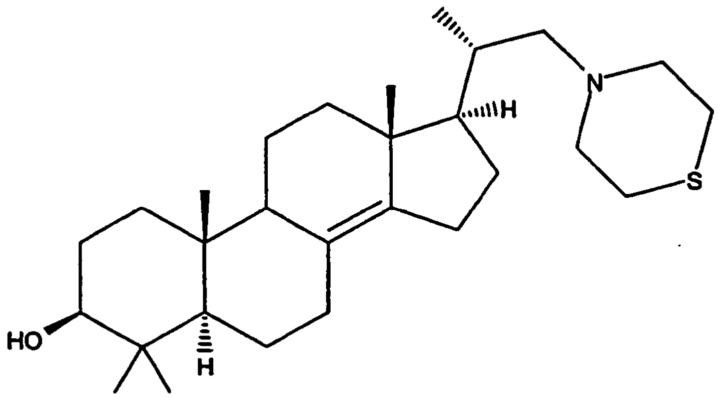

**Compound IB**

(20S)20-[(thiomorpholin-4-yl)methyl]-5α-pregna-8,14-dien-3-β-ol:

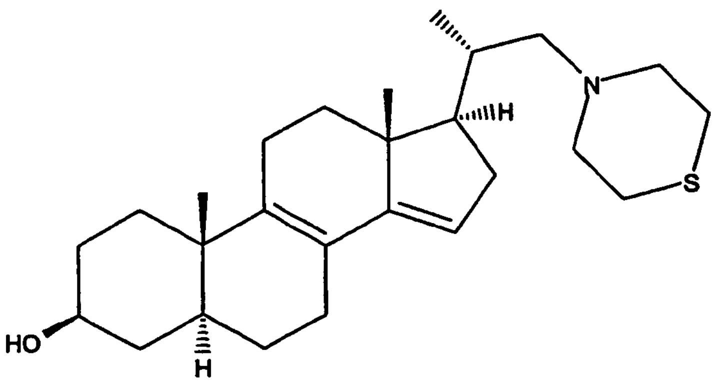

**Compound IC**

(20S)-20-[(thiomorpholin-4-yl)methy]-4,4-dimethyl-pregna-5,7-dien-3β-ol:

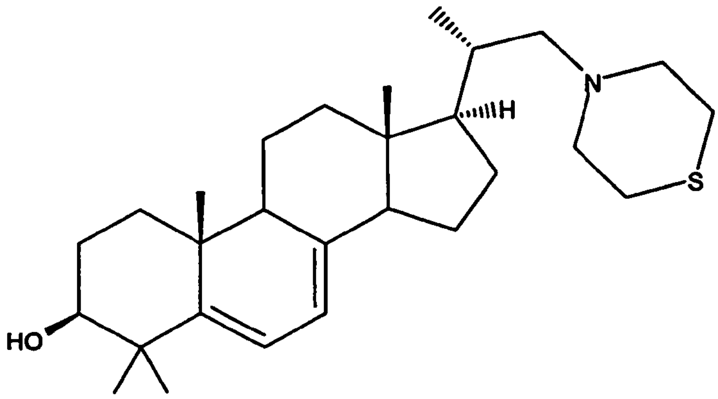

**Compound ID**

(20S)-20-[(thiomorpholin-4-yl)methyl]-5α-pregna-8(14)-en-3β-ol:

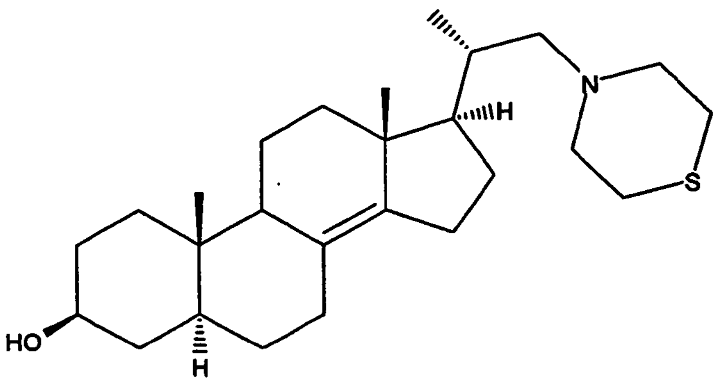

**Compound IE**

The most preferred thiomorpholino compound according to the present invention is -(20S)-20-[(thiomorpholin-4-yl)methyl]-4,4-dimethyl-5α-pregna-8,14 dien-3β-ol having chemical formula **IA**. Thus optimum performance with respect to induction of maturation in a follicular culture system is achieved with thiomorpholino derivatives if the steroid skeleton comprises a $\Delta^{8,14}$ double bond system and if $R^4$ and $R^{4'}$ are methyl (compound **IA**).

The novel steroid compounds have a number of chiral centers such that these compounds exist in several isomeric forms. All these isomeric forms are within the scope of the present invention unless otherwise described herein.

It has surprisingly been found that the compounds according to the present invention have a strong meiosis stimulating effect in oocytes, especially in CEOs, though these compounds are structurally highly different to sterol FF-MAS. In this respect the compounds of this invention are superior to this formerly described meiosis-regulating substance [*e.g.*: A.G. Byskov et al., Nature, 374, 559-562 (1995)]. Preferred compounds of general formula **I** are those, which induce the germinal vesicle breakdown by at least 40%, preferably at least 60% and especially at least 80% when tested in an oocyte test as described herein below.

The compounds according to the present invention are superior to the formerly described compounds in a second aspect: Whereas FF-MAS is not able to induce maturation in a follicle culture system, the compounds of the present invention are able to activate meiosis in this situation.

Compared to the compounds disclosed in WO 02/079220 A2 the thiomorpholino steroid compounds of the present invention exhibit an even stronger maturation-stimulating activity, especially in *in vitro* fertilization, and a higher successful retransfer rate into the tubes of a female after *in vitro* fertilization. Further the novel compounds exhibit superior properties with respect to the number of fetuses alive at the end of pregnancy if retransfer of the stimulated oocytes has been effected. Moreover the solubility of the compounds according to the present invention in water is better than the

solubility of the prior art compounds. It has also been found that growth of blastocysts is stimulated superiorly as could be shown in mice.

The foregoing advantages of the novel compounds are also true for the thiomorpholino compounds according to the present invention when they are compared to the most efficient compounds disclosed in WO 02/079200 A2, especially to (20S)-20-[(piperidin-1-yl)methyl]-4,4-dimethyl-5α-pregna-8,14-dien-3β-ol (compound no. 2 in this document).

For this reason the novel steroid compounds can *e.g.* be employed for *in vivo* use as well as for *non-in vivo* use, which especially comprises *in vitro* use. The steroid compounds are especially suitable for *in vitro* and for *in vivo* fertilization of mammals, especially of humans.

The outstanding properties of the novel compounds may be attributed to the combination of structural features in the compounds, *i.e.* primarily the position of double bonds in the steroid skeleton and the thiomorpholino group in the side chain linked to the $C^{17}$ carbon atom in the steroid skeleton via a methylen spacer and the $C^{20}$-$R^{20}$ group.

Preferably pharmaceutically acceptable compounds of the present invention are salts of steroid compounds of general formula **I**. Examples of these salts are listed in Journal of Pharmaceutical Science, 66, 2 et seq. (1977), which are hereby incorporated by reference. Examples of such salts include salts of organic acids such as of formic acid, fumaric acid, acetic acid, propionic acid, glycolic acid, lactic acid, pyruvic acid, oxalic acid, succinic acid, malic acid, tartaric acid, citric acid, benzoic acid, salicylic acid, methane sulfonic acid and the like. Suitable inorganic acids to form pharmaceutically acceptable salts include hydrochloric acid, hydrobromic acid, sulfuric acid, phosphoric acid and the like.

A further object of the present invention are pharmaceutical compositions comprising at least one thiomorpholino steroid compound of general formula **I** and at least one pharmaceutically acceptable excipient, selected from the group of excipients which are well known in the art. The excipient may *e.g.* be at least one carrier, diluent, absorption enhancer, preservative, buffer, agent for adjusting the osmotic pressure and rheology of the medicament if the pharmaceutical composition will be liquid, at least one surfactant, solvent, tablet disintegrating agent, micro capsules, filler, slip additive, colorant, flavour and other ingredient. These substances are conventionally used in the art. The thiomorpholino steroid compounds according to the present invention are preferably comprised in the pharmaceutical compositions in an effective amount

Examples for solid carriers are magnesium carbonate, magnesium stearate, dextrin, lactose, sugar, talkum, gelatin, pectin, starch, silica gel, tragacanth, methylcellulose, sodium carboxymethyl cellulose, low melting waxes and cacao butter.

Liquid compositions include sterile solutions, suspensions and emulsions, which may be administered *e.g.* orally by nasal administration or as an ointment. Such liquid compositions may also be suitable for injection or for use in connection with *ex vivo* or *in vivo* application. For oral administration the liquid may contain a pharmaceutically acceptable oil and/or lipophilic, surfactant and/or solvent which are miscible with water. In this connection reference is made to WO 97/21440 A1.

Liquid compositions may also contain other ingredients, which are conventionally used in the art, some of which are mentioned in the list above. Further a composition for transdermal administration of a compound of the present invention may be provided in the form of a patch. A composition for nasal administration may be provided in the form of a nasal spray in liquid or in powder form.

In order to enhance bioavailability of the thiomorpholino steroid compound these compounds may also be formulated as cyclodextrin chlatrates. For this purpose the compounds are compounded with α-, β- or γ-cyclodextrin or derivatives thereof.

Salves, ointments, lotions and other liquids to be administered externally must be in a condition such that the thiomorpholino steroid compounds of the present invention may be delivered to the subject in need of regulation of meiosis in sufficient quantity. For this purpose the medicament contains the above excipients for regulating the rheology of the medicament and other additives, further substances for enhancing skin permeation ability and protective skin substances such as conditioners and moisture regulators.

The medicament may also contain further active agents to enhance or regulate the effectiveness of the thiomorpholino steroid compounds or to produce other desired effects of the medicament.

For parenteral administration the steroid compounds may be dissolved or suspended in a pharmaceutically acceptable diluent Oils are very often used in combination with solvents, surfactants, suspension or emulsion agents, e.g. olive oil, peanut oil, soybean oil, caster oil and the like. For the preparation of an injectable medicament any liquid carrier may be employed. These liquids often also contain agents for the regulation of the viscosity thereof as well as agents for regulating isotonicity of the liquid.

The thiomorpholino steroid compound may further be administered as an injectable depot or as an implantate, which may *e.g.* be administered subcutanely, such that delayed release of the thiomorpholino steroid compounds is made possible. For this purpose various techniques may be employed, *e.g.* administration via a depot, which includes a membrane containing the active compound, or administration via a slowly dissolving depot. Implantates may *e.g.* contain biologically degradable polymers or synthetic silicones as inert material.

The dose of the thiomorpholino steroid compound to be used will be determined by a physician and will depend *i.a.* on the particular steroid compound employed, on the route of administration and on the purpose of the use. In general, the pharmaceutical compositions of the present invention are prepared by intimately bringing into association the active compound with the liquid or solid auxiliary ingredients and then, if necessary, shaping the product into the desired formulation.

Usually not more than 3000 mg, preferably not more than 350 mg, and in some preferred instances not more than 30 mg of the steroid compounds are to be administered to mammals, e.g. to humans, per day.

The present invention also relates to the use of the thiomorpholino steroid compounds of general formula **I** for the preparation of a composition being useful to regulate reproduction, e.g. meiosis. This composition is preferably applicable as a medicament.

The present invention further relates to a use of the novel thiomorpholino steroid compounds of general formula **I** to the preparation of a contraceptive or of a profertility drug.

The present invention further relates to the use of the thiomorpholino steroid compound of general formula **I** for non-*in vivo* use.

Regulation of reproduction, *e.g.* of meiosis, is used herein to indicate that the compounds according to the present invention are especially suitable to stimulate reproduction, *e.g.* meiosis, in mammal, especially in humans, of oocytes, such that these compounds which are agonistic analogues of a naturally occurring meiosis activating substance (FF-MAS), can be used in the treatment of infertility which is due to insufficient stimulation of meiosis in females and males.

The route of administration of compositions containing a compound of the present invention may be any route, which effectively transports the active steroid compound to its site of action.

Thus, when the steroid compounds are to be administered to a mammal, they are conveniently provided in the form of a pharmaceutical composition, which comprises at least one thiomorpholino steroid compound according to the present invention in connection with a pharmaceutically acceptable carrier. For oral use, such compositions are preferably in the form of tablets or capsules.

The thiomorpholino steroid compounds may be synthesized analogously with the preparation of known compounds. Hence, synthesis of the steroid compounds of formula **I** may follow the well established synthetic pathways described in the comprehensive sterol and steroid literature. The following literature may be used as the key source for synthesis: L.F. Fieser & M. Fieser: Steroids, Reinhold Publishing Corporation, N.Y., 1959; Rood's Chemistry of Carbon Compounds (ed.: S. Coffrey): Elsevier Publishing Company, 1971; and especially Dictionary of Steroids (eds.: R.A. Hill, D.N. Kirk, H.L.J. Makin and G.M. Murphy), Chapman & Hall, this literature hereby being incorporated by reference. The last one contains an extensive list of citations to the original papers covering the period up to 1990.

The present invention also specifically relates to a method for the preparation of (20S)-20-[(thiomorpholin-4-yl)methyl]-4,4-dimethyl-5α-pregna-8,14-dien-3β-ol (compound **IA**), the method comprising the following method steps:

a) starting from (20S)-20-hydroxymethyl-pregna-4-en-3-one;
b) introducing two alkyl groups in $C^4$ by alkylation;
c) reducing the keto group to a hydroxy group;
d) protecting the resulting hydroxy group with an acyl group, preferably with a benzoyl group;
e) introducing a $\Delta^7$ double bond by bromination/dehydrobromination;
f) isomerizing the dien $\Delta^{5,7}$ to the dien $\Delta^{8,14}$ by heating in the presence of acid;
g) oxidizing the 20-hydroxy group to an aldehyde group;
h) reductively aminizing the aldehyde group with thiomorpholine and removing the benzoyl group by reduction reaction.

The corresponding synthesis scheme of this first synthesis method is shown in Fig. 1 (Scheme 1). According to this, first the hydroxy group in the side chain of (20S)-20-hydroxymethyl-pregna-4-en-3-one 1 is protected as a silylether, e.g. as a triisopropylsilyl (TIPS) ether resulting in compound 2 (method step a). In order to produce compound 3 two methyl groups are introduced via alkylation with methyl iodide in the presence of a base like potassium *tert*-butoxide in $C^4$ of the steroid skeleton (method step b). In the next step the 3-keto group is reduced with a common reducing agent like lithium aluminiumhydride or sodium borohydride (method step c). The resulting alcohol 4 is then protected *e.g.* as a benzoate (compound 5; method step d). A second double bond is afterwards introduced via a bromination-dehydrobromination sequence (method step e). The resulting $\Delta^{5,7}$-dien system in compound 6 is then isomerized to the $\Delta^{8,14}$-dien system via heating in the presence of hydrochloric acid to obtain compound 7 (method step f). In this acid catalyzed step only the hydroxy group in the side chain is deprotected and compound 7 is obtained. As a result a selective oxidation of the hydroxy group in the side chain with Dess-Martin-Periodinane results in aldehyde 8 (method step g), which serves as an intermediate to introduce a thiomorpholine moiety in the side chain via reductive amination. For this purpose different reducing agents like sodium borohydride or tris-(acetoxy) borohydride may be used. After the reductive amination the deprotection of the 3-benzoate is done under reductive conditions with $LiAlH_4$ in a one pot procedure (method step

h). As a result the steroid compound I according to the present invention is obtained.

Instead of forming the benzoate in method step d) another protective acyl group may be introduced such as e.g. the acetyl group. If the acetate is formed intermediately instead of the benzoate deprotection of hydroxy in method step f) does not only take place in the side chain but also at $C^3$. In this case selective oxidation to the respective aldehyde must be performed cautiously in order to prevent oxidation of the hydroxy group at $C^3$. If the benzoate is formed instead in method step d) oxidation can only proceed at the hydroxy group in the side chain. Due to the fact that after method step d) the product obtained is crystallizing, if a benzoate protective group is used, easier purification of the intermediate 8 is possible. This furthermore enables to carry out less purification steps. Therefore formation of the benzoate is preferred.

As far as synthesis of steroid compounds without methyl groups at $C^4$ and/or with another double bond pattern in the steroid skeleton is concerned reference is made to WO 02/079220 A2, the respective disclosure thereof being incorporated by reference.

Examples are given to more detailedly describe the present invention.

**Example** 1: (20S)-20-[(thiomorpholin-4-yl)methyl]-4,4-dimethyl-5α-pregna-8,14-dien-3β-ol (compound **IA)**

**a)** (20S)-20-[((triisopropylsilyl)oxy)methyl]-pregna-4-en-3-one (Method step a)

To a solution of 30 g of (20S)-20-[(hydroxymethyl]-pregna-4-en-3-one and 13.5 g imidazole in 300 ml dichloromethane 26 ml of triisopropylsilylchloride were added dropwise at room temperature. The reaction mixture was stirred for 20 hours at the same temperature and then poured into water. The aqueous layer was extracted with ethyl acetate. The organic layers were combined, washed with brine, dried over sodium sulfate, filtered and concentrated under reduced pressure to give 45.4 g of crude (20S)-20-[((triisopropylsilyl)oxy)-methyl]-pregna-4-en-3-one as a brown oil, which was used without further purification.
MS (CI+): 487 (M + H)

b) (20S)-4,4-dimethyl-20-[((triisopropylsilyl)oxy)methyl]-pregna-5-en-3-one (Method step b)

A solution of 45.4 g of crude (20S)-20-[((triisopropylsilyl)oxy)methyl]-pregna-4-en-3-one in 320 ml tetrahydrofuran was added to a solution of 42.3 g potassium tert-butylate in 950 ml *tert*-butanol at a temperature of 50°C. The mixture was stirred for 10 minutes at the same temperature. Then 50 ml methyl iodide were added and stirring was continued for 1 hour. The reaction mixture was poured into water and extracted with ethyl acetate. The organic layers were combined, washed with brine, dried over sodium sulfate, filtered and concentrated under reduced pressure. The residue was purified by column chromatography to give 27.3 g (20S)-4,4-dimethyl-20-[((triisopropylsilyl)oxy)-methyl]-pregna-5-en-3-one as a pale yellow solid.
MS (CI+): 515 (M + H)

c) (20S)-4.4-dimethyl-20-[((triisopropylsill)oxy)methyl]-pregna-5-en-3β-ol (Method step c)

To a solution of 27.3 g (20S)-4,4-dimethyl-20-[((triisopropylsilyl)oxy)methyl]-pregna-5-en-3-one in 500 ml tetrahydrofuran 1.24 g of lithiumaluminum hydride were added cautiously in small portions at room temperature. The reaction mixture was stirred for one hour and then cooled to 0°C. 2.5 ml water, 2.5 ml of a 1 N sodium hydroxide solution and 7.5 ml of water were added successively. The mixture was filtered over celite. The filtrate was concentrated under reduced pressure. The residue was purified by column chromatography to give 18.2 g (20S)-4,4-dimethyl-20-[((triisopropylsilyl)oxy)methyl]-pregna-5-en-3β-ol as a pale yellow solid.
MS (CI+): 517 (M + H)

d) (20S)-4,4-dimethyl-20-[((triisopropylsilyl)oxy)methyl]-pregna-5-en-3β-ol acetate

(Method step d)

To a solution of 18.2 g (20S)-4,4-dimethyl-20-[((triisopropylsilyl)oxy)methyl]-pregna-5-en-3β-ol in 175 ml pyridine 6.24 ml of acetic anhydride were added at room temperature. The reaction mixture was stirred for 20 hours and then poured into an ice/hydrochloric acid mixture. This was extracted with ethyl acetate. The organic layers were combined, washed with brine, dried over sodium sulfate, filtered and concentrated under reduced pressure to give 16.2 g (20S)-4,4-dimethyl-20-[((triisopropylsiiyl)oxy)methyl]-pregna-5-en-3-one acetate as a white solid, which was used without further purification.
MS (CI+): 559 (M + H)

e) (20S)-4,4-dimethyl-20-[((triisopropylsilyl)oxy)methyl]-pregna-5,7-dien-3β-ol acetate

(Method step e)

To a solution of 16.2 g (20S)-4,4-dimethyl-20-[((triisopropylsilyl)oxy)methyl]-pregna-5-en-3β-ol acetate in a mixture of 100 ml benzene and 100 ml hexane 4.93 g 1,3-dibrom-5,5-dimethyl-hydantoin were added in portions at 70°C. After 30 minutes the mixture was cooled to 0°C and filtered. The filtrate was evaporated *in vacuo.*

To the resulting residue 160 ml toluene and 7.8 ml 2,4,6-trimethylpyridine were added. The mixture was refluxed for 2.5 hours. After cooling the reaction mixture was washed with 1 N hydrochloric acid, saturated sodium bicarbonate solution and brine. The organic layer was dried over sodium sulfate, filtered and evaporated *in vacuo.* The residue was purified by column chromatography to give 12.5 g (20S)-4,4-dimethyl-20-[((triisopropylsilyl)oxy)methyl]-pregna-5,7-dien-3β-ol acetate as a white solid.
MS (Cl+): 557 (M + H)

f) (20S)-4,4,20-trimethyl-gregna-8.14-dien-3β,21-diol

(Method step f)

A mixture of 16.1 g (20S)-4,4-dimethyl-20-[((triisopropylsilyl)oxy)methyl]-pregna-5,7-dien-3β-ol acetate, 210 ml ethanol, 28 ml benzene and 28 ml concentrated hydrochloric acid was refluxed for 6 hours. After cooling the mixture was poured into saturated sodium bicarbonate solution, extracted with ethyl acetate and washed with brine. The organic layer was dried over sodium sulfate, filtered and concentrated under reduced pressure. The residue was recrystallized from dichloromethane and methanol to give 4.48 g (20S)-21-hydroxy-4,4,20-trimethyl-pregna-8,14-dien-3β-ol.
MS (EI+): 358 (M)

g) (20S)-3β-hydroxy-4,4,20-trimethyl-pregna-8,14-dien-21-al (Method step g)

To a solution of 1 g (20S)-4,4,20-trimethyl-pregna-8,14-dien-3β,21-diol in 10 ml dichloromethane 5.4 ml of a 0.5 M Dess-Martin-Periodinane solution were added at room temperature. The mixture was stirred for one hour, poured into saturated sodium bicarbonate solution, extracted with ethyl acetate and washed with brine. The organic layer was dried over sodium sulfate, filtered and concentrated under reduced pressure. The residue was purified by column chromatography to give 230 mg (20S)-3β-hydroxy-4,4,20-trimethyl-pregna-8,14-dien-21-al as a white solid.
MS (EI+): 356 (M)

h) (20S)-20-[(thiomorpholin-4-yl)methyl]-4,4-dimethyl-5α-pregna-8,14-dien-3β-ol

(Method step h)

38 mg sodium tris(acetoxy)borohydride were added to a solution of 42 mg (20S)-3p-hydroxy-4,4,20-trimethyl-pregna-8,14-dien-21-al and 20 μl thiomorpholine in 3 ml tetrahydrofuran at room temperature. The mixture was stirred for two hours, poured into saturated sodium bicarbonate solution, extracted with ethyl acetate and washed with brine. The organic layer was dried over sodium sulfate, filtered and concentrated under reduced pressure. The residue was purified by column chromatography to give 15 mg (20S)-20-[(thiomorpholine-4-yl)methyl]-4,4-dimethyl-5α-pregna-8,14-dien-3β-ol as a white solid.
MS (EI+): 443 (M)
NMR spectra were in accordance with the structure thereof.
The structure was also confirmed by X-ray structural analysis.

**Example 2:** Reaction according to reaction scheme 1, Fig. 1

The reactions of example 1 were repeated producing the benzoate analogue of intermediate compounds **5, 6, 7** and **8**. For this purpose the reaction conditions in the several method steps were modified as follows:

| | |
|---|---|
| Method step a): | TIPSCl, imidazole, $CH_2Cl_2$, room temperature, 4 hours reaction time |
| Method step b): | potassium *tert*-butylate, methyl iodide, tert-butanol, room temperature, 30 minutes reaction time |
| Method step c): | $LiAlH_4$, tetrahydrofuran, room temperature, 30 minutes reaction time |
| Method step d): | benzoyl chloride, pyridine, 0°C, 1 hour reaction time; crystallization; 52% over 4 steps |

(continued)

| | |
|---|---|
| Method step e): | 1,3-dibromo-5,5-dimethyl-imidazolidine-2,4-dione, benzene, hexane, 70°C, 30 minutes reaction time; then 2,4,6-trimethylpyridine, toluene, reflux, 2 hours reaction time |
| Method step f): | HCl, ethanol, reflux, 4 hours reaction time, chromatography; 70% over 2 steps |
| Method step g): | Dess-Martin-Periodinane, $CH_2Cl_2$, room temperature |
| Method step h): | Thiomorpholine, $NaBH(OAc)_3$, tetrahydrofuran, room temperature, 6 hours reaction time; then reaction with $LiAlH_4$, room temperature; 18 hours reaction time, chromatography and crystallization from ethanol (2x), yield: 19% over 2 steps and purification (purity > 93%). |

The overall yield in this reaction sequence was calculated to be 6.9% over eight steps.

The detailed reaction sequence and reaction conditions are outlined herein after

a - d) <u>(20S)-4,4-dimethyl-20-[((triisopropylsilyl)oxy)methyl]-pregna-5-en-3β-ol benzoate (compound 5, see scheme Fig. 1)</u>

(Method steps a, b, c and d)

This compound was synthesized in analogy to the acetate route described under Example 1, method steps a, b, c and d, and is also described in Organic Letters, 5, 1837-1839 (2003). Only one crystallization as a purification step was done after the benzoylation. The overall yield for the steps a, b, c and d was 52%.

e) <u>(20S)-4,4-dimethyl-20-[((triisoropylsilyl)oxy)methyl]-pregna-5,7-dien-3β-ol benzoate (compound 6, see scheme Fig. 1)</u>

(Method step e)

To a solution of 30.0 g (20S)-4,4-dimethyl-20-[((triisopropylsilyl)oxy)methyl]-pregna-5-en-3β-ol benzoate in a mixture of 160 ml benzene and 160 ml hexane 9.81 g 1,3-dibrom-5,5-dimethyl-hydantoin were added in portions at 70°C. After 30 minutes the mixture was cooled to 0°C and filtered. The filtrate was evaporated *in vacuo.*

To the resulting residue 280 ml toluene and 12.7 mi 2,4,6-trimethylpyridine were added. The mixture was refluxed for 2.5 hours. After cooling the reaction mixture was washed with 0.5 N hydrochloric acid, saturated sodium bicarbonate solution and brine. The organic layer was dried over sodium sulfate, filtered and evaporated *in vacuo* to give 31.8 g of crude (20S)-4,4-dimethyl-20-[((triisopropyl)oxy)methyl]-pregna-5,7-dien-3β-ol benzoate, which was used without further purification.

f) <u>(20S)-3β-benzoyloxy-4,4,20-trimethyl-pregna-8,14-dien-21-ol (compound 7. see scheme Fig.1)</u>

(Method step f)

A mixture of 31.8 g (20S)-4,4-dimethyl-20-[((triisopropyl)oxy)methyl]-pregna-5,7-dien-3β-ol benzoate, 467 ml ethanol, 67 ml benzene and 67 ml concentrated hydrochloric acid was refluxed for 5 hours. After cooling the mixture was poured into saturated sodium bicarbonate solution, extracted with dichloromethane and washed with brine. The organic layer was dried over sodium sulfate, filtered and concentrated under reduced pressure. The residue was purified by column chromatography to give 15.7 g (20S)-3β-benzoytoxy-4,4,20-trimethyl-pregna-8,14-dien-21-ol.

g) <u>(20S)-3β-enzoyloxy-4.4.2-methyl-pregna-8,14-dien-21-al (compound 8, see scheme Fig. 1)</u>

(Method step g)

To a solution of 17.6 g (20S)-3β-benzoyloxy-4,4,20-trimethyl-pregna-8,14-dien-21-ol in 470 ml tetrahydrofurane and 4.1 ml pyridine 17.3 ml of Dess-Martin-Periodinane were added at 0°C. The mixture was stirred for 30 minutes, warmed to room temperature, poured into pH 7 citrate buffer, extracted with dichloromethane and washed with brine. The organic layer was dried over sodium sulfate, filtered and concentrated under reduced pressure to give 17.6 g of crude (20S)-3β-benzoyloxy-4,4,20-trimethyl-pregna-8,14-dien-21-al, which was used without further purification.

h) (20S)-20-[(thiomorpholin-4-yl)methyl]-4,4-dimethyl-5α-pregna-8,14-dien-3β-ol (compound I, see scheme Fig.1)

(Method step h)

3.9 g sodium tris(acetoxy)borohydride were added to a solution of 8.8 g of crude (20S)-3β-benzoyloxy-4,4,20-trimethyl-pregna-8,14-dien-21-al and 2.7 ml thiomorpholine in 400 ml tetrahydrofuran at room temperature. The mixture was stirred for two hours, then 10.56 g of lithium aluminium hydride were added in portions, quenched with NaOH and water, extracted with ethyl acetate and washed with brine. The organic layer was dried over sodium sulfate, filtered and concentrated under reduced pressure. The residue was purified by column chromatography and two consecutive crystallizations from ethanol to give 1.67 g (20S)-20-[(thiomorpholin-4-yl)methyl]-4,4,-dimethyl-5α-pregna-8,14-dien-3β-ol as a white solid (1.43 g + 0.24 g, two crystallization crops were obtained).

All compounds were characterized by $^1$H-NMR and MS. Final proof of the structure of (20S)-20-[(thiomorpholine-4-yl)methyl]-4,4-dimethyl-5α-pregna-8,14-dien-3β-ol (compound **IA**) was achieved by X-ray structural analysis.

Chemical and physical-chemical characterization and formulation (solubility):

It could be shown that the introduction of a nitrogen containing side chain improves the solubility of the compounds in water (measured by turbimetry).

The solubility of FF-MAS in water was found to be only < 0.1 mg/l. The solubility of (20S)-20-[(thiomorpholin-4-yl)methyl]-4,4-dimethyl-5α-pregna-8,14-dien-3β-ol (compound **IA**) in water was measured to be ~ 4 mg/l.

Oocyte *in vitro* maturation and fertilization (biological test systems):

a) Cumulus Enclosed Oocyte Assay:

Detailed description of the test:

Activation of MI arrested oocytes can be assessed by the disappearance of the germinal vesicle (GV). The disappearance of the GV, called germinal vesicle breakdown (GVB) is followed by the extrusion of the first polar body (PB) (C. Grøndahl, J.L. Ottesen, M. Lessi, P. Faarup, A. Murray, FC Grønvald, C. Hegele-Hartung, I. Ahnfelt-Rønne, "Meiosis-activating sterol promotes resumption of meiosis in mouse oocytes cultured in vitro in contrast to related oxysterols", Biol. Reprod., 58,1297-1302 (1998); C. Hegele-Hartung, J. Kuhnke, M. Lessl, C. Grøndahl, J. Ottesen, H.M. Beier, S. Eisner, U. Eichenlaub-Ritter, "Nuclear and cytoplasmic maturation of mouse oocytes after treatment with synthetic meiosis-activating sterol in vitro", Biol. Reprod., 61, 1362-1372 (1999)).

Cumulus Enclosed Oocytes (CEOs) were obtained from immature female mice (C57BU6J x DBA/2J F1) weighing 13 -16 grams, that were kept under controlled temperature (20 - 22°C), light (lights on 06:00 -18:00) and relative humidity (50 - 70%). The mice received an intra-peritoneal injection of 0.2 ml gonadotropins containing 20 IU FSH. 48 hours later the animals were killed by cervical dislocation. The ovaries were dissected out and the oocytes were isolated in Hx-medium (see below) under a stereomicroscope by manual rupture of the follicles using a pair of 27-gauge needles. Cumulus enclosed oocytes displaying an intact germinal vesicle were placed in α-minimum essential medium (α-MEM without ribonucleosides supplemented with 8 mg/ml Human Serum Albumin (HSA), 0,23 mM pyruvate, 2 mM glutamine, 100 IU/ml penicillin and 100 μg/ml streptomycin). To maintain the oocytes in the germinal vesicle stage this medium was supplemented with 3 mM hypoxanthine, designated as Hx-medium.

Dilutions of (20S)-20-[(thiomorpholin-4-y1)methyl]-4,4-dimethyl-5α-pregna-8,14-dien-3β-ol (compound **IA**) were prepared from a stock solution containing 1 mg/ml dissolved in ethanol. Addition of ethanol (3.8 μl/ml) to controls did not affect the control level. The CEOs (35 - 45 CEOs in 0.4 ml Hx-medium) were cultured in a humidified atmosphere of 5% $CO_2$ in air for 24 hours at 37°C. One control well (identical medium with no addition of test compound) was always cultured simultaneously with test wells.

By the end of the culture period the number of CEOs with germical vesicle (GV), germinal vesicle breakdown (GVB) and polar bodies (PB), respectively, were counted. The % GVB, defined as the percentage of CEOs undergoing GVB per total number of CEOs in that well, was calculated as:

$$\% \text{ GVB} = (\text{number of GVB} + \text{number of PB} / \text{total number of CEOs}) \times 100$$

The % PB was defined as the percentage of CEOs displaying one extruded polar body per total number of CEOs in that well.

Results:

Fig. 2 shows the meiosis activation of (20S)-20-[(thiomorpholin-4-yl)methyl]-4,4-dimethyl-5a-pregna-8,14-dien-3β-ol (compound **IA**) in CEOs monitored by GVB and PB (n = 5; *p < 0.05). The novel compound **IA** significantly improved meiosis activation at 1 μM (p < 0.01; n = 10), at 3 μM (p < 0.05; n = 7) and 10 μM (p < 0.001; n = 6).

The same experiments were repeated with the other novel compounds:

**IB:** (20S)-20-[(thiomorpholin-4-yl)methyl]-4,4-dimethyl-5α-pregna-8(14)-en-β-ol
**IC:** (20S)-20-[(thiomorpholin-4-yt)methyl]-5α-pregna-8,14-dien-3β-ol
**ID:** (20S)-20-[(thiomorpholin-4-yl)methyl]-4,4-dimethyl-pregna-5,7-dien-3β-ol and
**IE:** (20S)-20-[(thiomorpholin-4-yl)methyl]-5α-pregna-8(14)-en-3β-ol.

The results of the activation of meiosis in the presence of hypoxanthine (Hx) in CEOs are given in Table 1. For each compound two experiments were performed and the results compared to Hx-medium as a control and FF-MAS at a concentration of 10 μM.

In addition concentration dependence of meiosis activation in the presence of Hx in CEOs was determined using compound **IE**, which was, in addition to compound **IA**, the most efficient compound tested. The concentration dependence of meiosis activation as compared to that of Hx-medium, of FF-MAS and of (20S)-20-[(piperidin-1-yl)methyl]-4,4-dimethyl-5α-pregna-8,14-dien-3β-ol (disclosed in WO 02/079220 A2 as compound no. 2) is given in Table 2.

b) *In vitro* fertilization (IVF)

To investigate the effect of (20S)-20-[(thiomorphotin-4-yl)methyl]-4,4-dimethyl-5α-pregna-8,14-dien-3β-ol (compound **IA**) on the IVF rate CEOs were *in vitro* matured in the presence or absence of this novel compound with subsequent IVF. FF-MAS and (20S)-20-[(piperidin-1-yl)methyl]-4,4-dimethyl-5α-pregna-8,14-dien-3β-ol (disclosed in WO 02/079220 A2 as the compound no. 2) served as control compounds. Since media for *in vitro* maturation in a clinical setting would not contain hypoxanthine, this novel compound was not used in any media, which resulted in nearly 100% meiosis activation of the oocytes due to spontaneous meiotic maturation. Changes in the subsequent IVF are therefore probably due to effects of the test compounds on cytoplasmic maturation.

Immature female mice (C57BU6J x DBA/2J F1) in the age of 21 - 24 days were used as oocyte donors. Animals were given an *i.p.* injection of 10 IU pregnant mare serum gonadotropin (PMSG) to induce follicular growth. Animals were killed by cervical dislocation 48 hours later and the ovaries were dissected out. As sperm donors eight week old male mice (C57BL/6J x DBA/2J F1) were used.

Oocytes were isolated from the ovaries by manual rupture of the follicles using a pair of 27-gauge needles. Spherical naked oocytes (NkO) and CEOs displaying an intact GV were selected and placed in α-minimum essential medium (α-MEM without ribonucleosides, Gibco BRL, Gaithersburg; Cat. No. 22561) supplemented with 8 mg/ml human serum albumin (HSA), 0.23 mM pyruvate, 2 mM glutamine, 100 IU/ml penicillin and 100 μg/ml streptomycin.

The oocytes were rinsed three times in culture medium without oil overlay in 4-well multidishes containing 0.4 ml of the respective oocyte culture medium. The oocytes were cultured at 37°C in a humidified atmosphere of 5% $CO_2$ in air in the presence of (20S)-20-[(thiomorpholin-4-yl)methyl]-4,4-dimethyl-5α-pregna-8,14-dien-3β-ol (compound **IA**), FF-MAS and (20S)-20-[(piperidin-1-yl)methyl]-4,4-dimethyl-5α-pregna-8,14-dien-3β-ol, which latter are compounds of comparison.

The oocyte culture medium was supplemented with 6.4% Fetal Bovine Serum (FBS). Solutions of novel compound **IA** and of the two compounds in comparison as mentioned were prepared from a stock solution containing 1 mg/ml of compound dissolved in absolute ethanol. The control group was cultured with a corresponding amount of ethanol.

After a culture time of 18 -19 hours oocytes were further processed for *in vitro* fertilization. Only oocytes that exhibited either GVB or a PB were defined as *"in vitro* matured oocytes". Oocytes were briefly washed without any test compound and subsequently transferred to the insemination dishes prepared in advance, which contained approximately 600.000 capacitated sperm cells per 500 μl IVF medium (obtained from the epididymides of male mice). The dishes were then incubated under defined gas conditions (5% $CO_2$ in air) at 37°C in a modified α-MEM IVF medium supplemented with 8 mg/ml HSA, 0.23 mM pyruvate, 100 IU peniciitin/mi and 100 μg streptomycin/ml as described above for oocyte maturation.

Examination of the oocytes was carried out 22 - 24 hours after insemination in order to check fertilization and to record the number of pronucleus (PN) and 2-cell stage embryos. The fertilization rate was determined as the number of oocytes that had reached the PN or 2-cell embryo stage, relative to the total number inseminated.

Results:

Fig. 3 shows higher fertilization rates of mouse oocytes with the novel compound **IA** and the two compounds as mentioned, (20S)-20-[(piperidin-1-yl)methyl]-4,4-dimethyl-5a-pregna-8,14-dien-3β-ol (compound no. 2) or FF-MAS compared to vehicle control (*$p < 0.05$; ***$p < 0.001$; $n = 18$). Fig. 3a gives the values in percent of total oocytes used in the experiment (approximately 100 oocytes per group in each group for each experiment). Fig. 3b gives the data as stimulation factor normalized to the vehicle group.

Retransfer Study:

*In vitro* fertilization was performed as described above for the IVF experiment.

One day before the retransfer female recipient mice (foster mothers) in pro-estrus cycle state were mated with vasectomized males in order to trigger pseudogestation. One day later a vaginal check was performed. Animals with a positive, sperm-free vaginal plug (successful mating) entered the study. This day was designated day 0 of gestation (= day 0 after the sterile copulation, day 0 p.c.). Eighteen 2-cell embryos were transferred into the right tube of the foster mothers on day 0 after the sterile copulation under anesthesia. The contralateral tube remained unused (check for pseudogestation). The foster mothers were sacrified on day 19 of gestation. The fetuses were removed and examined. The ovaries and uteri were removed immediately to record the number of implantation sites, viable and dead fetuses and resorptions. Apparently non-pregnant uteri were placed in a 10% aqueous solution of ammonium sulfide for about 10 min to stain possible implantation sites in the endometrium. The viable fetuses were individually weighed and their sex determined by inspection of their gonads.

In a fertility trial 269 mice were randomized into 6 groups and implanted with 7 -10 *in vitro* fertilized ova. The *in vitro* fertilization took place in the presence of vehicle (group 1) or of a substance (groups 2 to 6). The groups were distributed as given in Table 3.

The fertility raising effect of the substances in comparison to the vehicle control was of main interest. Moreover, an improvement compared to the substance FF-MAS was also investigated.

Variables of primary interest are the number of implantation spots and the number of viable fetuses.

Methods:

The data for implantation spots as well as for viable fetuses have to be considered to have binary character, *i.e.* it can be expressed in rates or in a number of successes and failures with respect to the number of transferred ova. Therefore, methods as outlined by D. Collett: "Modelling Binary Data", 2nd edition, Chapman & Hall / CRC, Boca Raton, constitute appropriate approaches to the data analysis.

Since the odds for a success in one group as compared to the odds for a success in another group lend itself for the interpretation of these data, the analysis focuses on the computation of odds ratios. The odds ratio of one group as compared to the other is defined as:

$$\text{odds ratio (group 1, group 2)} = or_{1,2} = \text{odds (group 1) / odds (group 2)}$$

where the odds of a group can be estimated by the number of successes divided by the number of failures in that group.

To reach statements about the significance of any findings, 95% confidence-intervals for the odds ratios are computed as:

$$[\exp(\log(or_{1,2}) / 1.96 \cdot se(\log(or_{1,2}))) \; ; \; \exp(\log(or_{1,2}) + 1.96 \cdot se(\log(or_{1,2})))]$$

wherein $se(\log(or_{1,2}))$ is the standard error of the log odds ratio (see D. Collett (2003) for the corresponding formula).

The odds of one group can be claimed to be significantly larger than the odds in the other group, if the lower confidence limit is larger than 1.

Comparisons to the Vehicle Control:

As a basis for the computation of odds ratios, the number of successes (*i.e.,* the number of implantation spots of number of viable fetuses) and failures (*i.e..* the number of transferred ova minus the number of implantation spots or

number of viable fetuses) are computed for all groups. Table 4 shows these numbers and the resulting odds for the six groups.

The odds ratios of the five groups in comparison to the vehicle control and their confidence intervals are shown in Table 5.

For the number of implantation spots, compound **IA** shows a significant improvement of odds in both doses. This novel compound in 1 µM increases the odds of a successful implantation by 51%, while 10 µM lead to a 55% better chance of a successful implantation. Neither FF-MAS nor the compound no. 2 ((Z0S)-20-[(piperidin-1-yl)methyl]-4,4-dimethyl-5α-pregna-8,14-dien-3β-ol) show a significant improvement of odds.

The odds for a success in terms of viable fetuses are increased by 70% by compound **IA** in the lower doses. None of the other groups display significant differences to the vehicle control.

Comparisons to FF-MAS:

The odds ratios of groups 3 to 6 in comparison to FF-MAS (group 2) and their confidence intervals are shown in Table 6.

Since all confidence intervals include the value 1, no significant difference between the four substance groups and FF-MAS can be concluded.

Fig. 4 shows the implantation rate per transferred 2-cell embryos, reaching higher numbers for treatment with FF-MAS (10 µM) and the novel compounds **IA** at 1 µM and 10 µM. The numbers of live pubs are given in Fig. 5. Again numbers of live pubs were recorded after treatment with FF-MAS and the novel compound **IA** (1 µM and 10 µM). Pregnancy rates for each group are shown in Fig. 6.

Noticeably the novel compound **IA** shows a beneficial effect on both the 2-cell and blastocyst rate already at a concentration of 0.1 µM which tends to be even higher -at a concentration of 1µM.

It is understood that the examples and embodiments described herein are for illustrative purpose only and that various modifications and changes in light thereof as well as combinations of features described in this application will be suggested to persons skilled in the art and are to be included within the scope of the appended claims. All publications, patents and patent applications cited herein are hereby incorporated by reference.

Table 1: Activation of meiosis in the presence of Hx in CEOs

| Meiosis Activation [%] | | | |
|---|---|---|---|
| | Compound, 10 µM | Hx (control) | FF-MAS, 10 µM |
| Compound **IB** | - | 30 | 48 |
| | 15 | 24 | 32 |
| Compound **IC** | 64 | 4 | 20 |
| | 69 | 20 | 16 |
| Compound **ID** | 89 | 50 | 71 |
| | 58 | 32 | 56 |
| Compound **IE** | 96 | 20 | 60 |
| | 80 | 12 | 28 |

Table 2: Activation of meiosis in the presence of Hx in CEOs

| Meiosis Activation [%] | | | | | |
|---|---|---|---|---|---|
| Compound **IE** | | | Hx (control) | FF-MAS | Compound No. 2 *) |
| 1µM | 3µM | 10µM | | 10 µM | 10µM |
| 44 | 24 | 60 | 17 | 24 | 48 |
| *) (20S)-20-[(piperidin-1-yl)methyl]-4,4-dimethyl-5α-pregna-8,14-dien-3β-ol | | | | | |

Table 3:

| Group Number | Description |
|---|---|
| 1 | Vehicle Control |
| 2 | FF-MAS, 10 μM |
| 3 | Compound No. 2 *), 1 μM |
| 4 | Compound No 2 *), 10 μM |
| 5 | Compound **IA**, 1 μM |
| 6 | Compound **IA**, 10M |
| *) (20S)-20-[(piperidin-1-yl)methyl]-4,4-dimethyl-5α-pregna-8,14-dien-3β-ol | |

Table 4: Successes, Failures and Odds per Group

| Variable | Group Number | Description | Success | Failures | Odds |
|---|---|---|---|---|---|
| No. of Implantation Spots | 1 | Vehicle Control | 86 | 344 | 0.25 |
| | 2 | FF-MAS, 10 μM | 100 | 335 | 0.30 |
| | 3 | Compound No. 2*), 1 μM 1 μM | 97 | 357 | 0.27 |
| | 4 | Compound No. 2*). 10 μM | 90 | 332 | 0.27 |
| | 5 | Compound **IA**, 1 μM | 120 | 317 | 0.38 |
| | 6 | Compound **IA**, 10 μM | 123 | 317 | 0.39 |
| No. of Viable Fetuses | 1 | Vehicle Control | 31 | 399 | 0.08 |
| | 2 | FF-MAS, 10 μM | 45 | 390 | 0.12 |
| | 3 | Compound No. 2*). 1 μM | 32 | 422 | 0.08 |
| | 4 | Compound No. 2, 10 μM | 37 | 385 | 0.10 |
| | 5 | Compound **IA**, 1 μM | 51 | 386 | 0.13 |
| | 6 | Compound **IA**, 10 μM | 40 | 400 | 0.10 |
| *) (20S)-20-[(piperidin-1-yl)methyl]-4,4-dimethyl-5α-pregna-8,14-dien-3β-ol | | | | | |

Table 5: Odds Ratios and Confidence Limits for Comparison to Vehicle Control

| Variable | Group Number | Description | | 95%- Confidence Limits | |
|---|---|---|---|---|---|
| | | | Odds Ratio | Lower | Upper |
| No. of Implantation Spots | 2 | FF-MAS, 10 μM | 1.19 | 0.86 | 1.65 |
| | 3 | Compound No. 2*), 1 μM | 1.09 | 0.78 | 1.51 |
| | 4 | Compound No. 2*), 10 μM | 1.08 | 0.78 | 1.51 |
| | 5 | Compound **IA,** 1μM | 1.51 | 1.10 | 2.08 |
| | 6 | Compound **IA,** 10 μM | 1.55 | 1.13 | 2.13 |

(continued)

| Variable | Group Number | Description | | 95%- Confidence Limits | |
|---|---|---|---|---|---|
| | | | Odds Ratio | Lower | Upper |
| No. of Viable Fetuses | 2 | FF-MAS, 10 μM | 1.49 | 0.92 | 2.40 |
| | 3 | Compound No. 2*). 1 μM | 0.98 | 0.58 | 1.63 |
| | 4 | Compound No.2 10 μM | 1.24 | 0.75 | 2.03 |
| | 5 | Compound **IA**, 1 μM | 1.70 | 1.07 | 2.71 |
| | 6 | Compound **IA**. 10 μM | 1.29 | 0.79 | 2.10 |
| *) (20S)-20-[(piperidin-1-yl)methyl]-4,4-dimethyl-5α-pregna-8,14-dien-3β-ol | | | | | |

Table 6: Odds Ratios and Confidence Limits for Comparison to FF-MAS

| Variable | Group Number | Description | | 95%- Confidence Limits | |
|---|---|---|---|---|---|
| | | | Odds Ratio | Lower | Upper |
| No. of Implantation Spots | 3 | Compound No.2*), 1 μM | 0.91 | 0.66 | 1.25 |
| | 4 | Compound No. 2*), 10μM | 0.91 | 0.66 | 1.25 |
| | 5 | Compound **IA**, 1μM | 1.27 | 0.93 | 1.72 |
| | 6 | Compound **IA,** 10μM | 1.30 | 0.96 | 1.76 |
| No. of Viable Fetuses | 3 | Compound No.2*), 1 μM | 0.66 | 0.41 | 1.06 |
| | 4 | Compound No.2*), 10 μM | 0.83 | 0.53 | 1.32 |
| | 5 | Compound **IA**, 1 μM | 1.15 | 0.75 | 1.75 |
| | 6 | Compound **IA,** 10 μM | 0.87 | 0.55 | 1.36 |
| *) (20S)-20-[(piperidin-1-yl)methyl]-4,4-dimethyl-5α-pregna-8,14-dien-3β-ol | | | | | |

## Claims

1. A thiomorpholino steroid compound of general formula **I**

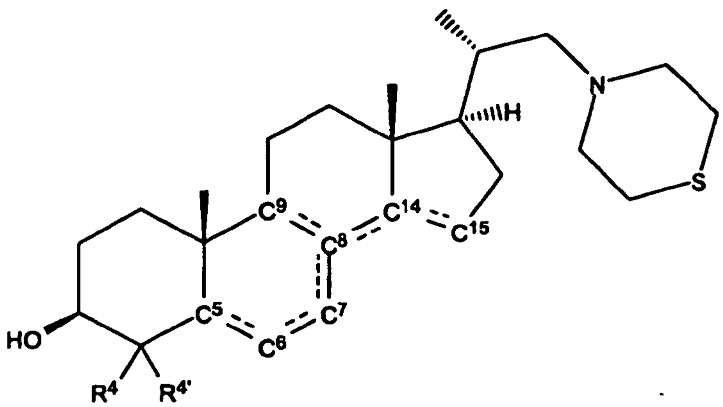

**I**

wherein in the moiety **I'** of compound **I**

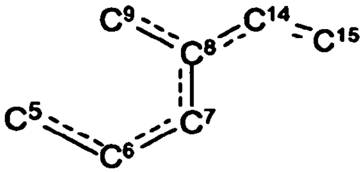

**I'**

each bond between between $C^5$ and $C^6$, between $C^6$ and $C^7$, between $C^7$ and $C^8$, between $C^8$ and $C^9$, between $C^8$ and $C^{14}$ and between $C^{14}$ and $C^{15}$, independently, is a single bond or a double bond, at least one of these bonds being a double bond, with the proviso that there is no double bond in the steroid skeleton exclusively between $C^5$ and $C^6$, and
wherein
$R^4$ and $R^{4'}$ independently, are selected from the group, comprising hydrogen and methyl.

**2.** The steroid compound according to claim 1, wherein in the moiety with general formula **I'** one double bond is present between $C^8$ and $C^{14}$ or two double bonds are present between $C^8$ and $C^9$ and between $C^{14}$ and $C^{15}$ or two double bonds are present between $C^5$ and $C^6$ and between $C^7$ and $C^8$.

**3.** The steroid compound according to any one of claims 1 and 2, being selected from the group comprising:

(20S)20-[(thiomorpholin-4-yl)methyl]-4,4-dimethyl-5α-pregna-8,14-dien-3β-ol:

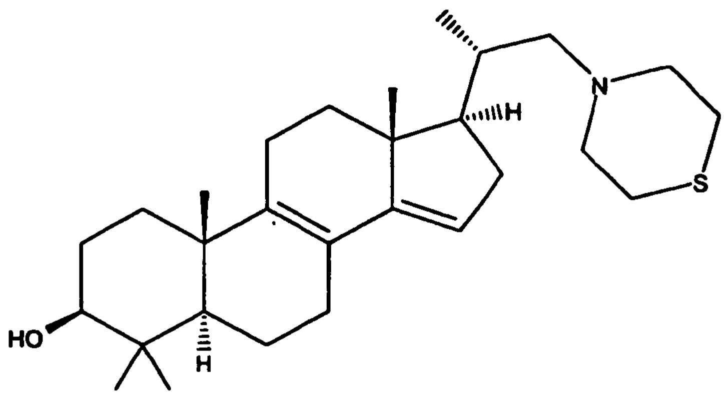

**IA**

(20S)-20-[(thiomorpholin-4-yl)methyl]-4.4-dimethyl-5α-pregna-8(14)-en- 3β-ol:

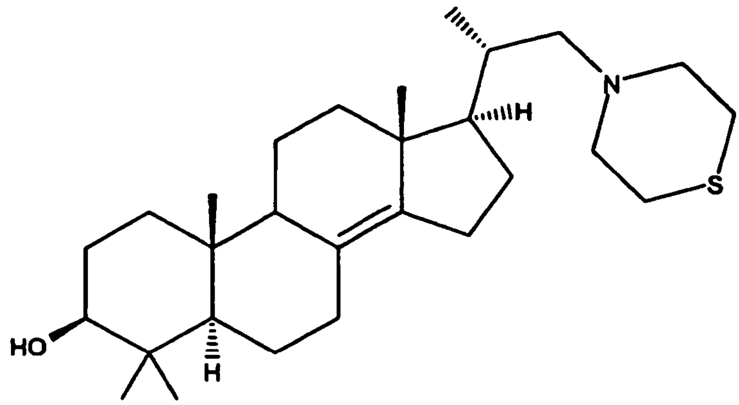

**IB**

(20S)20-[(thiomorpholin-4-yl)methyl]-5α-pregna-8,14-dien-3β-ol:

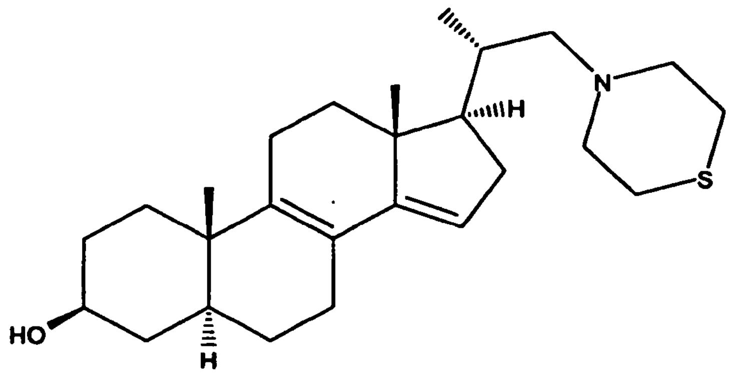

**IC**

(20S)-20-[(thiomorpholin-4-yl)methyl]-4,4-dimethyl-pregna-5,7-dien-3 β-ol

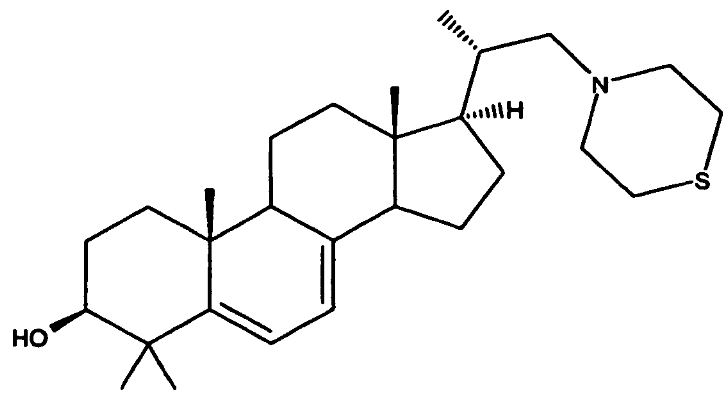

**ID**

(20S)-20-[(thiomorpholin-4-yl)methyl]-5α-pregna-8(14)-en-3β-ol:

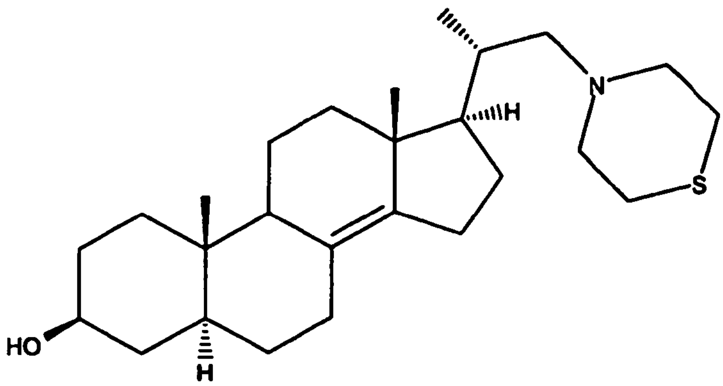

**IE.**

4. A pharmaceutical composition comprising at least one thiomorpholino steroid compound of general formula **I** according to any one of claims 1-3 and at least one pharmaceutically acceptable excipient.

5. The pharmaceutical composition according to claim 4, wherein the steroid compound of general formula **I** is comprised in an effective amount.

6. A use of the thiomorpholino steroid compound of general formula **I** according to any one of claims 1 - 3 to the preparation of a pharmaceutical composition being useful to regulate reproduction, especially meiosis.

7. The use according to claim 6 for non-*in vivo* use.

8. A use of the thiomorpholino steroid compound of general formula **I** according to any one of claims 1 - 3 to the preparation of a contraceptive or of a profertility drug.

9. A method for the preparation of (20S)-20-[(thiomorpholin-4-yi)methyl]-4.4-dimethyl-5α-pregna-8,14-dien-3β-ol, comprising

a) starting from (20S)-20-hydroxymethyl-pregna-4-en-3-one;
b) introducing two alkyl groups in $C^4$ by alkylation;
c) reducing the keto group to a hydroxy group;
d) protecting the resulting hydroxy group with an acyl group;
e) introducing a $\Delta^7$ double bond by bromination/dehydrobromination;
f) isomerizing the dien $\Delta^{5,7}$ to the dien $\Delta^{8,14}$ by heating in the presence of acid;
g) oxidizing the 17-hydroxy group to an aldehyde group;
h) reductively aminizing the aldehyde group with thiomorpholine and removing the acyl group by reduction reaction

10. The method according to claim 9, wherein the acyl group is a benzoate group

**Patentansprüche**

1. Thiomorpholinosteroidverbindungen der allgemeinen Formel **I**

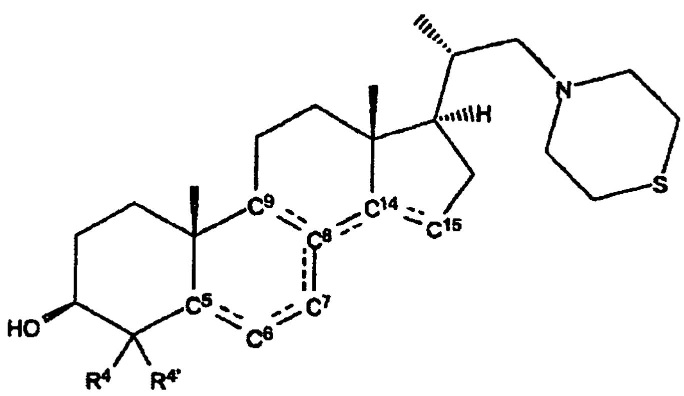

**I**

wobei in der Gruppe I' der Verbindung **I**

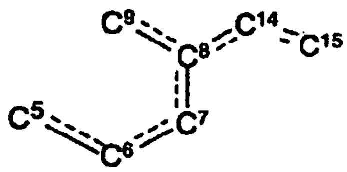

**I'**

die Bindungen zwischen $C^5$ und $C^6$, zwischen $C^6$ und $C^7$, zwischen $C^7$ und $C^8$, zwischen $C^8$ und $C^9$, zwischen $C^8$ und $C^{14}$ und zwischen $C^{14}$ und $C^{15}$ jeweils unabhängig voneinander für eine Einfachbindung oder eine Doppelbindung stehen, wobei wenigstens eine dieser Bindungen eine Doppelbindung ist, mit der Maßgabe, daß sich in dem Steroidgerüst keine Doppelbindung ausschließlich zwischen $C^5$ und $C^6$ befindet und
wobei
$R^4$ und $R^{4'}$ unabhängig voneinander aus der aus Wasserstoff und Methyl bestehenden Gruppe ausgewählt sind.

**2.** Steroidverbindungen nach Anspruch 1, wobei in der Gruppe mit der allgemeinen Formel **I'** eine Doppelbindung zwischen $C^8$ und $C^{14}$ vorhanden ist oder zwei Doppelbindungen zwischen $C^8$ und $C^9$ und zwischen $C^{14}$ und $C^{15}$ vorhanden sind oder zwei Doppelbindungen zwischen $C^5$ und $C^6$ und zwischen $C^7$ und $C^8$ vorhanden sind.

**3.** Steroidverbindungen nach Anspruch 1 oder 2, ausgewählt aus der Gruppe bestehend aus:

(20S)-20[(Thiomorpholin-4-yl)methyl]-4,4-dimethyl-5α-pregna-8,14-dien-3β-ol:

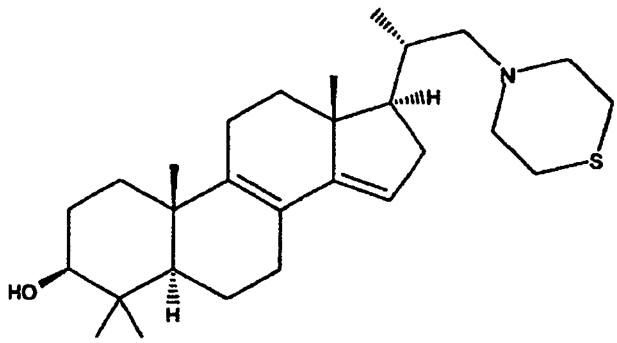

IA

(20S)-20[(Thiomorpholin-4-yl)methyl]-4,4-dimethyl-5α-pregna-8(14)-en-3β-ol:

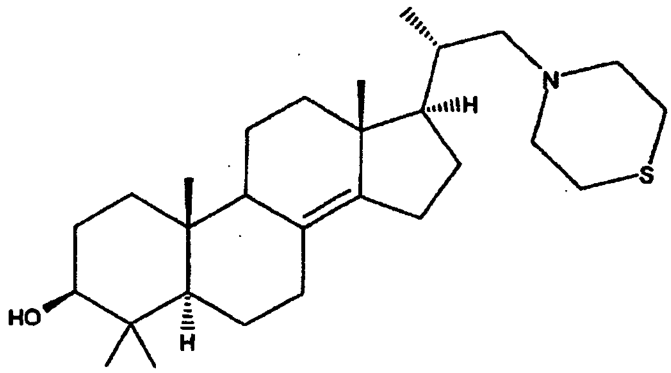

IB

(20S)-20[(Thiomorpholin-4-yl)methyl]-5α-pregna-8,14-dien-3β-ol:

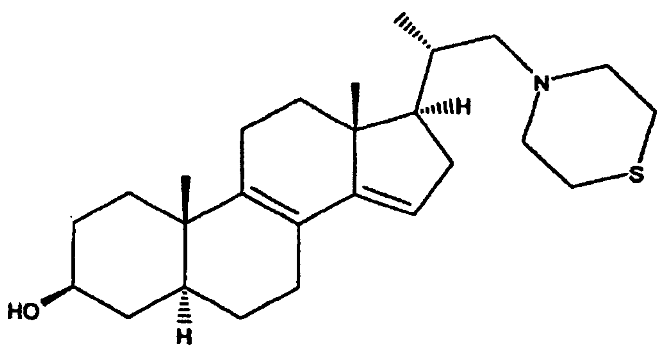

IC

(20S)-20[(Thiomorpholin-4-yl)methyl]-4,4-dimethylpregna-5,7-dien-3β-ol:

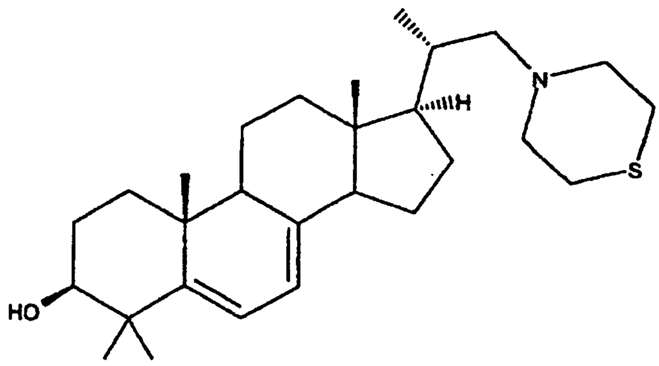

ID

(20S)-20[(Thiomorpholin-4-yl)methyl]-5α-pregna-8(14)-en-3β-ol:

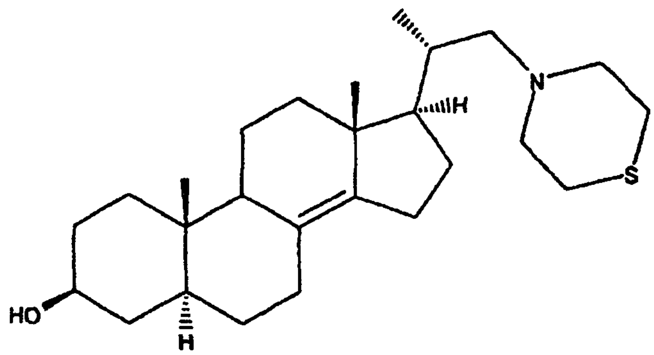

IE.

**4.** Pharmazeutische Zusammensetzung, enthaltend wenigstens eine Thiomorpholinosteroidverbindung der allgemeinen Formel **I** nach einem der Ansprüche 1-3 und wenigstens einen pharmazeutisch unbedenklichen Exzipienten.

**5.** Pharmazeutische Zusammensetzung nach Anspruch 4, wobei die Steroidverbindung der allgemeinen Formel **I** in einer wirksamen Menge enthalten ist.

**6.** Verwendung einer Thiomorpholinosteroidverbindung der allgemeinen Formel **I** nach einem der Ansprüche 1-3 zur Herstellung einer pharmazeutischen Zusammensetzung, die sich zur Steuerung der Reproduktion, insbesondere der Meiose, eignet.

**7.** Verwendung nach Anspruch 6 zur nicht-in-vivo-Verwendung.

**8.** Verwendung einer Thiomorpholinosteroidverbindung der allgemeinen Formel **I** nach einem der Ansprüche 1-3 zur Herstellung eines empfängnisverhütenden Mittels oder eines Arzneimittels zur Erhöhung der Fruchtbarkeit.

**9.** Verfahren zur Herstellung von (20S)-20-[(Thiomorpholin-4-yl)methyl]-4,4-dimethyl-5a-pregna-8,14-dien-3β-ol, bei dem man

a) von (20S)-20-Hydroxymethylpregna-4-en-3-on ausgeht;
b) durch Alkylierung zwei Alkylgruppen in $C^4$ einführt;
c) die Ketogruppe zu einer Hydroxylgruppe reduziert;
d) die auf diese Weise erhaltene Hydroxylgruppe mit einer Acylgruppe schützt;
e) durch Brominierung/Dehydrobrominierung eine $\Delta^7$- Doppelbindung einführt;
f) das Dien $\Delta^{5,7}$ durch Erhitzen in Gegenwart von Säure zum Dien $\Delta^{8,14}$ isomerisiert;

g) die 17-Hydroxylgruppe zu einer Aldehydgruppe oxidiert;
h) die Aldehydgruppe mit Thiomorpholin reduktiv aminiert und die Acylgruppe durch eine Reduktionsreaktion entfernt.

**10.** Verfahren nach Anspruch 9, wobei es sich bei der Acylgruppe um eine Benzoatgruppe handelt.

**Revendications**

**1.** Composé thiomorpholinostéroïde de formule générale **I**

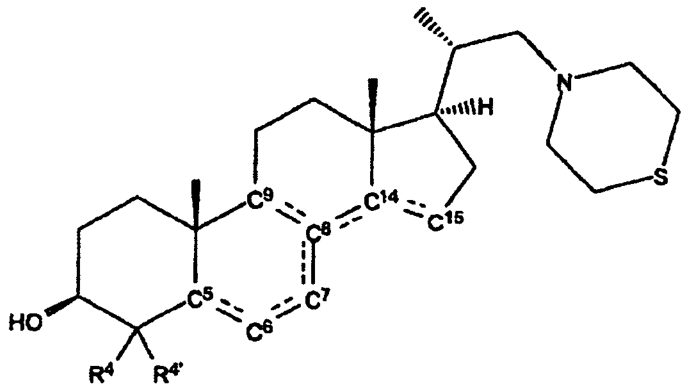

**I**

dans laquelle, dans le motif **I'** du composé **I**

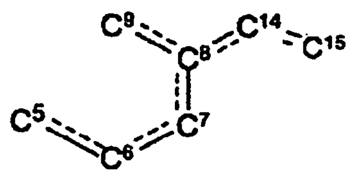

**I'**

chaque liaison entre $C^5$ et $C^6$ entre $C^6$ et $C^7$, entre $C^7$ et $C^8$, entre $C^8$ et $C^9$, entre $C^8$ et $C^{14}$ et entre $C^{14}$ et $C^{15}$, indépendamment, est une simple liaison ou une double liaison, au moins l'une de ces liaisons étant une double liaison, à condition qu'il n'y ait pas de double liaison dans le squelette stéroïde exclusivement entre $C^5$ et $C^6$, et dans laquelle
$R^4$ et $R^{4'}$ , indépendamment, sont choisis dans le groupe comprenant hydrogène et méthyle.

**2.** Composé stéroïde selon la revendication 1, **caractérisé en ce que**, dans le motif de formule générale **I',** une double liaison est présente entre $C^8$ et $C^{14}$ ou deux doubles liaisons sont présentes entre $C^8$ et $C^9$ et entre $C^{14}$ et $C^{15}$, ou deux doubles liaisons sont présentes entre $C^5$ et $C^6$ et entre $C^7$ et $C^8$.

**3.** Composé stéroïde selon l'une quelconque des revendications 1 et 2, **caractérisé en ce qu'**il est choisi dans le groupe comprenant :

le (20S)-20[(thiomorpholin-4-yl)méthyl]-4,4-diméthyl-5α-prégna-8,14-dién-3β-ol :

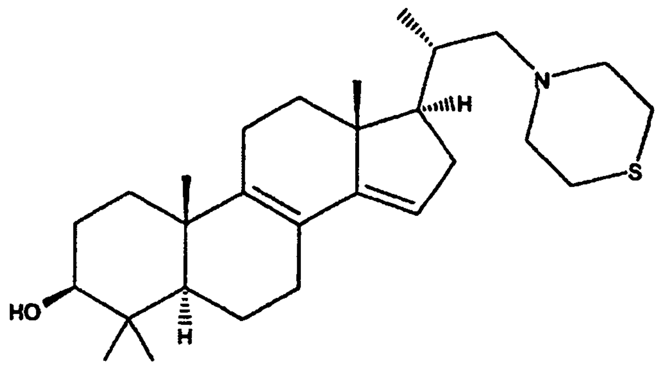

IA

le (20S)-20[(thiomorpholin-4-yl)méthyl]-4,4-diméthyl-5α-prégna-8(14)-én-3β-ol :

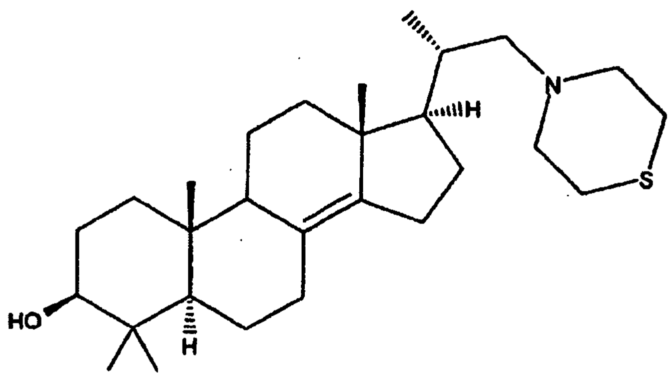

IB

le (20S)-20[(thiomorpholin-4-yl)méthyl]-5α-prégna-8,14-dién-3β-ol :

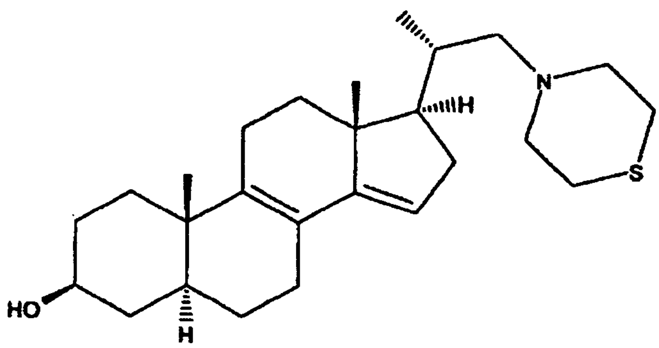

IC

le (20S)-20[(thiomorpholin-4-yl)méthyl]-4,4-diméthylprégna-5,7-dién-3β-ol :

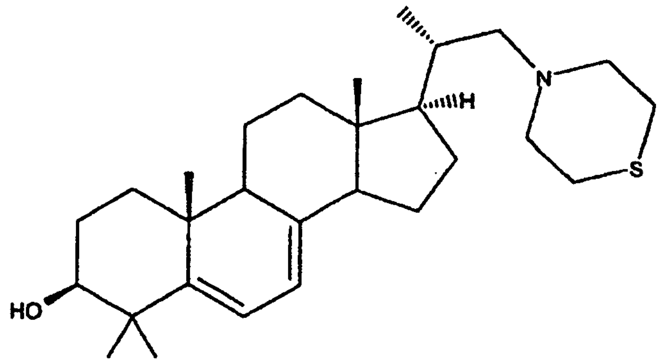

**ID**

le (20S)-20[(thiomorpholin-4-yl)méthyl]-5α-prégna-8(14)-én-3β-ol :

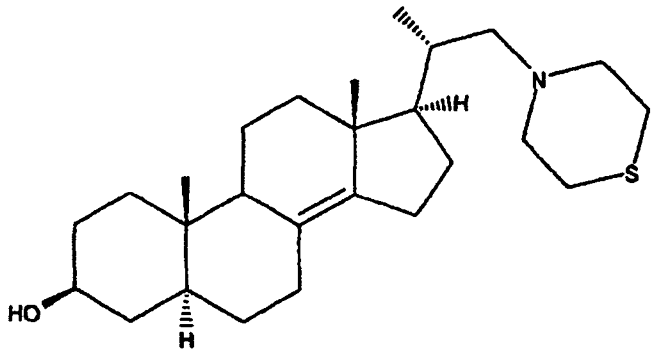

**IE.**

4. Composition pharmaceutique comprenant au moins un composé thiomorpholinostéroïde de formule générale **I,** selon l'une quelconque des revendications 1 à 3, et au moins un excipient pharmaceutiquement acceptable.

5. Composition pharmaceutique selon la revendication 4, **caractérisée en ce que** le composé stéroïde de formule générale **I** est présent en une quantité efficace.

6. Utilisation du composé thiomorpholinostéroïde de formule générale **I** selon l'une quelconque des revendications 1 à 3, pour la préparation d'une composition pharmaceutique utile pour réguler la reproduction, notamment la méiose.

7. Utilisation selon la revendication 6, pour une utilisation non *in vivo.*

8. Utilisation du composé thiomorpholinostéroïde de formule générale **I** selon l'une quelconque des revendications 1 à 3, pour la préparation d'un contraceptif ou d'un médicament contre la stérilité.

9. Méthode de préparation de (20S)-20[(thiomorpholin-4-yl)méthyl]-4,4-diméthyl-5α-prégna-8,14-dién-3β-ol, comprenant :

   a) à partir de (20S)-20-hydroxyméthylprégna-4-én-3-one ;
   b) l'introduction de deux groupements alkyle dans $C^4$ par alkylation ;
   c) la réduction du groupement céto en un groupement hydroxy ;
   d) la protection du groupement hydroxy résultant avec un groupement acyle ;
   e) l'introduction d'une double liaison $\Delta^7$ par bromation/déshydrobromation ;
   f) l'isomérisation du diène $\Delta^{5,7}$ pour obtenir le diène $\Delta^{8,14}$ par chauffage en présence d'un acide ;

g) l'oxydation du groupement 17-hydroxy en un groupement aldéhyde ;
h) l'amination réductrice du groupement aldéhyde avec de la thiomorpholine et l'élimination du groupement acyle par une réaction de réduction.

**10.** Méthode selon la revendication 9, **caractérisée en ce que** le groupement acyle est un groupement benzoate.

Scheme 1

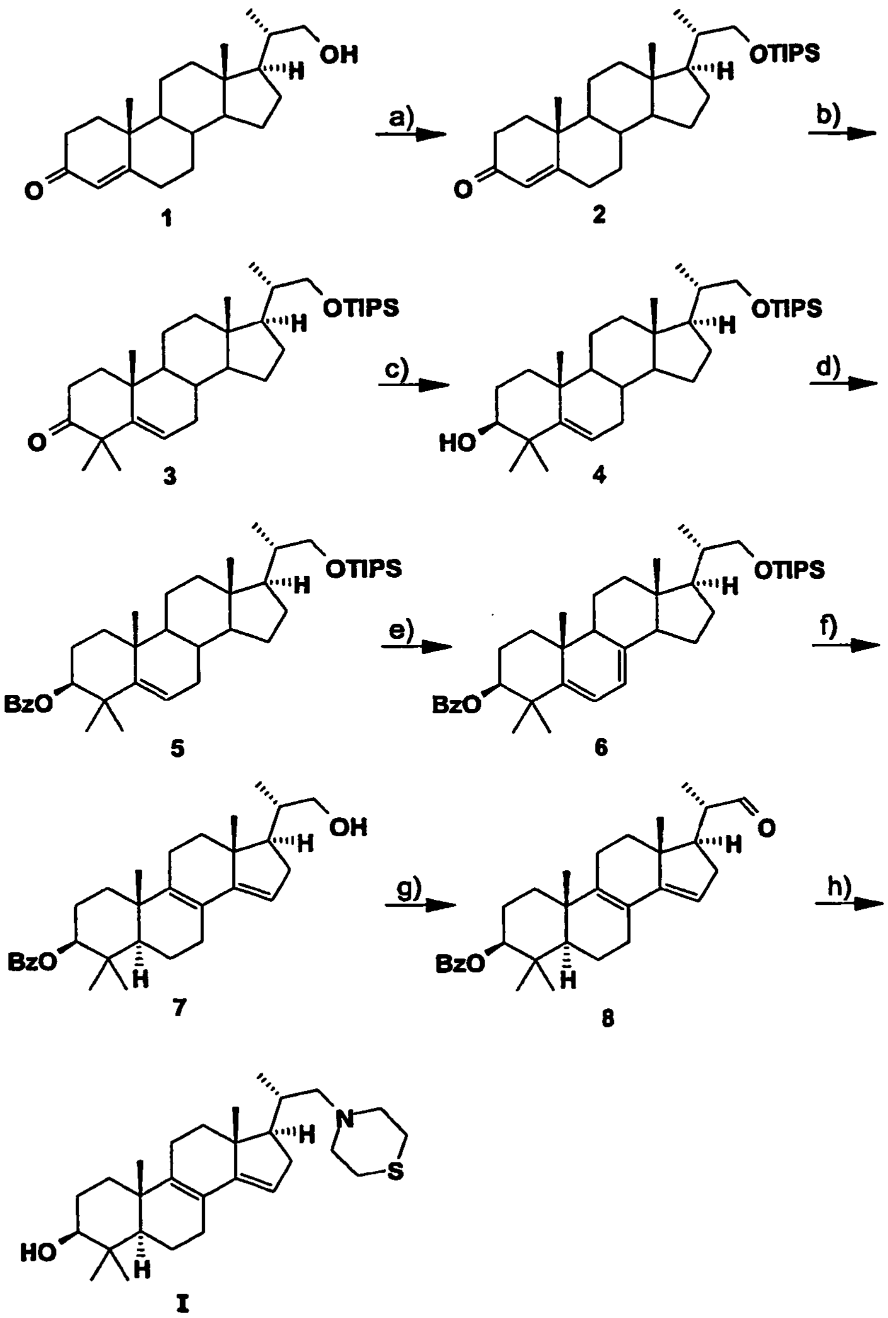


1

2

3

4

5

6

7

8

I

**Fig. 1**

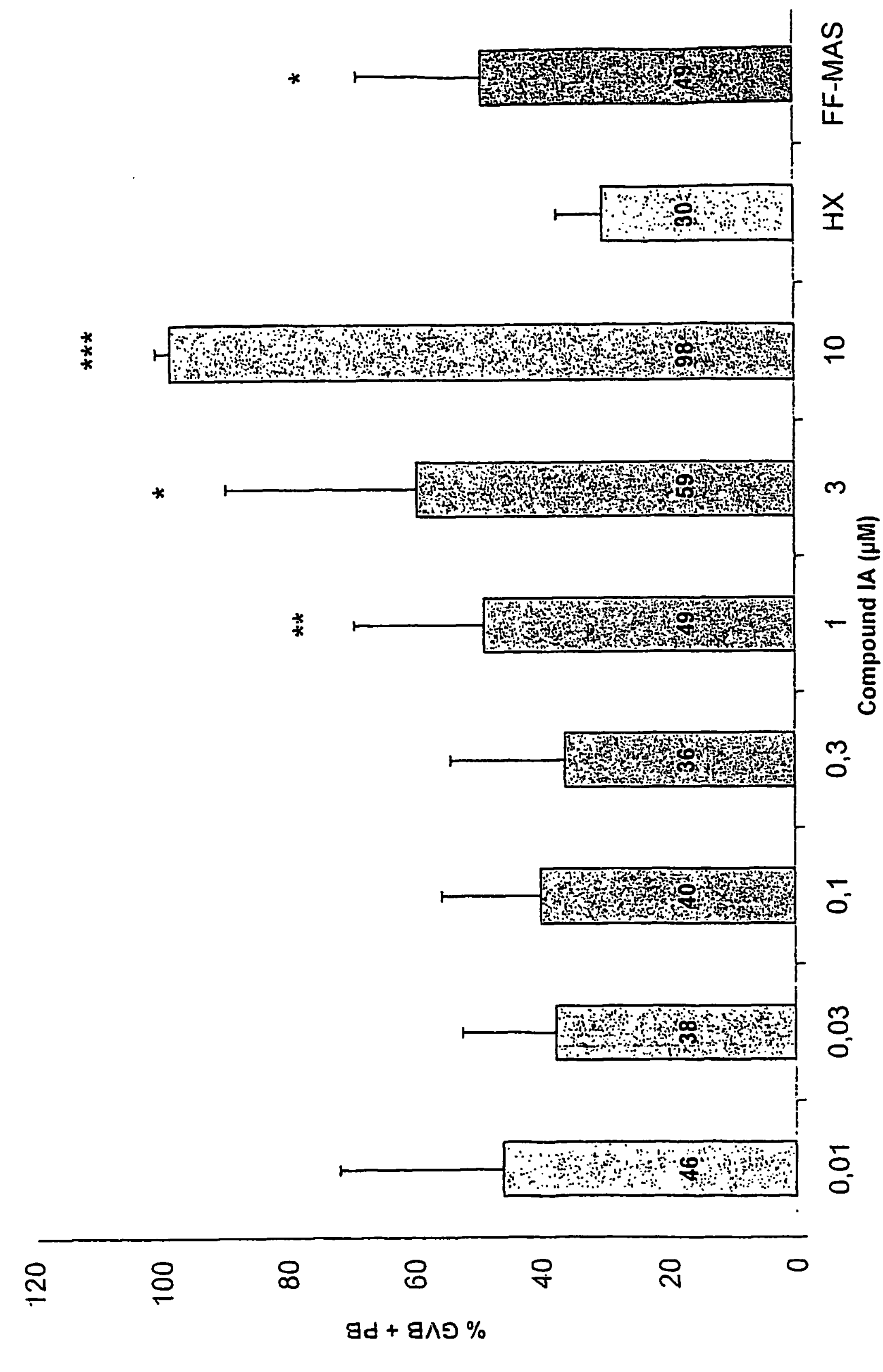

% GVB + PB
0
20
40
60
80
100
120
Compound IA (µM)
0,01
0,03
0,1
0,3
1
3
10
HX
FF-MAS
46
38
40
36
49
59
98
30
49
**
*
***
*


Fig. 2

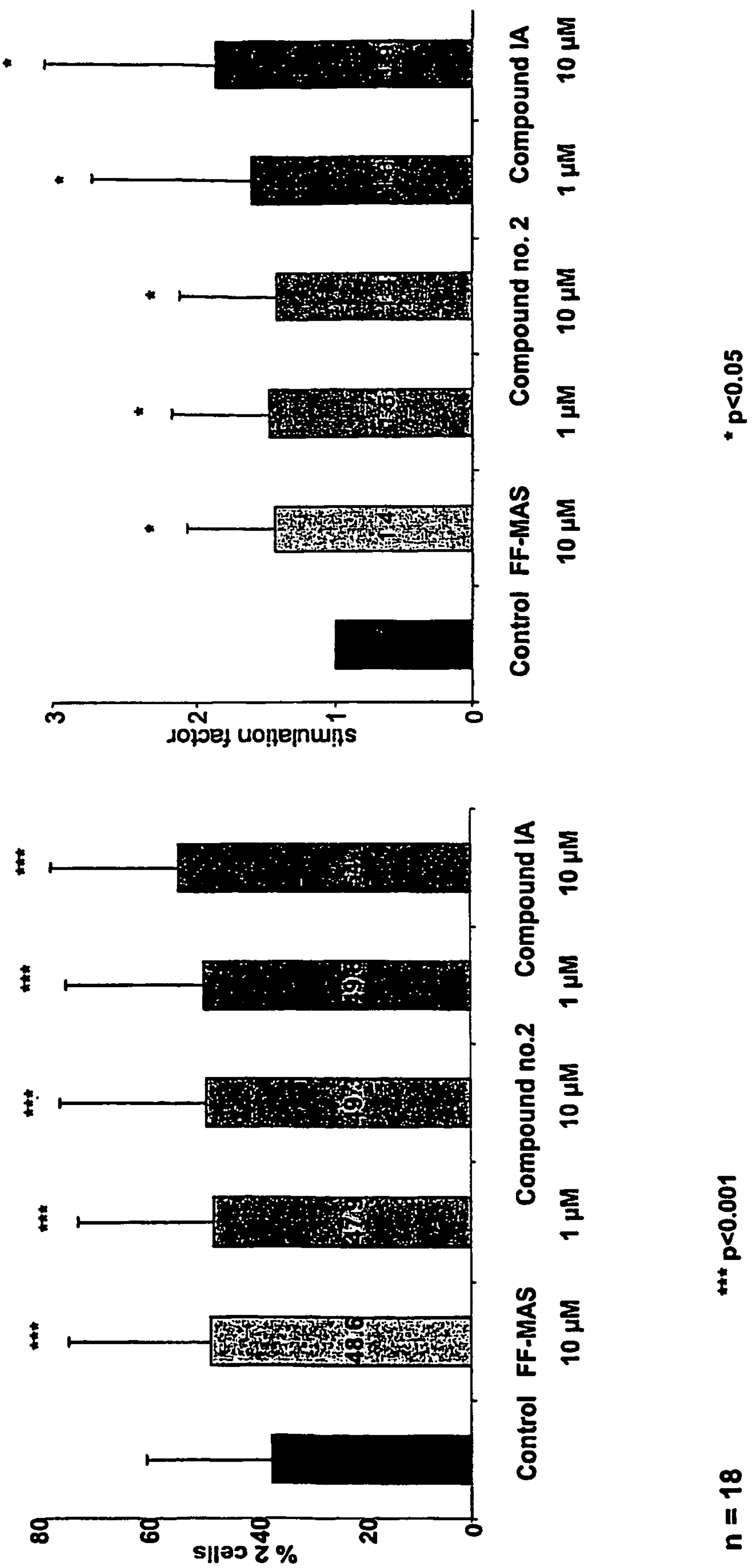
% 2 cells
0
20
40
60
80
Control
FF-MAS 10 µM
Compound no.2 1 µM
Compound no.2 10 µM
Compound IA 1 µM
Compound IA 10 µM
48.6
***
*** p<0.001
n = 18
stimulation factor
0
1
2
3
*
* p<0.05

3a

3b

Fig. 3

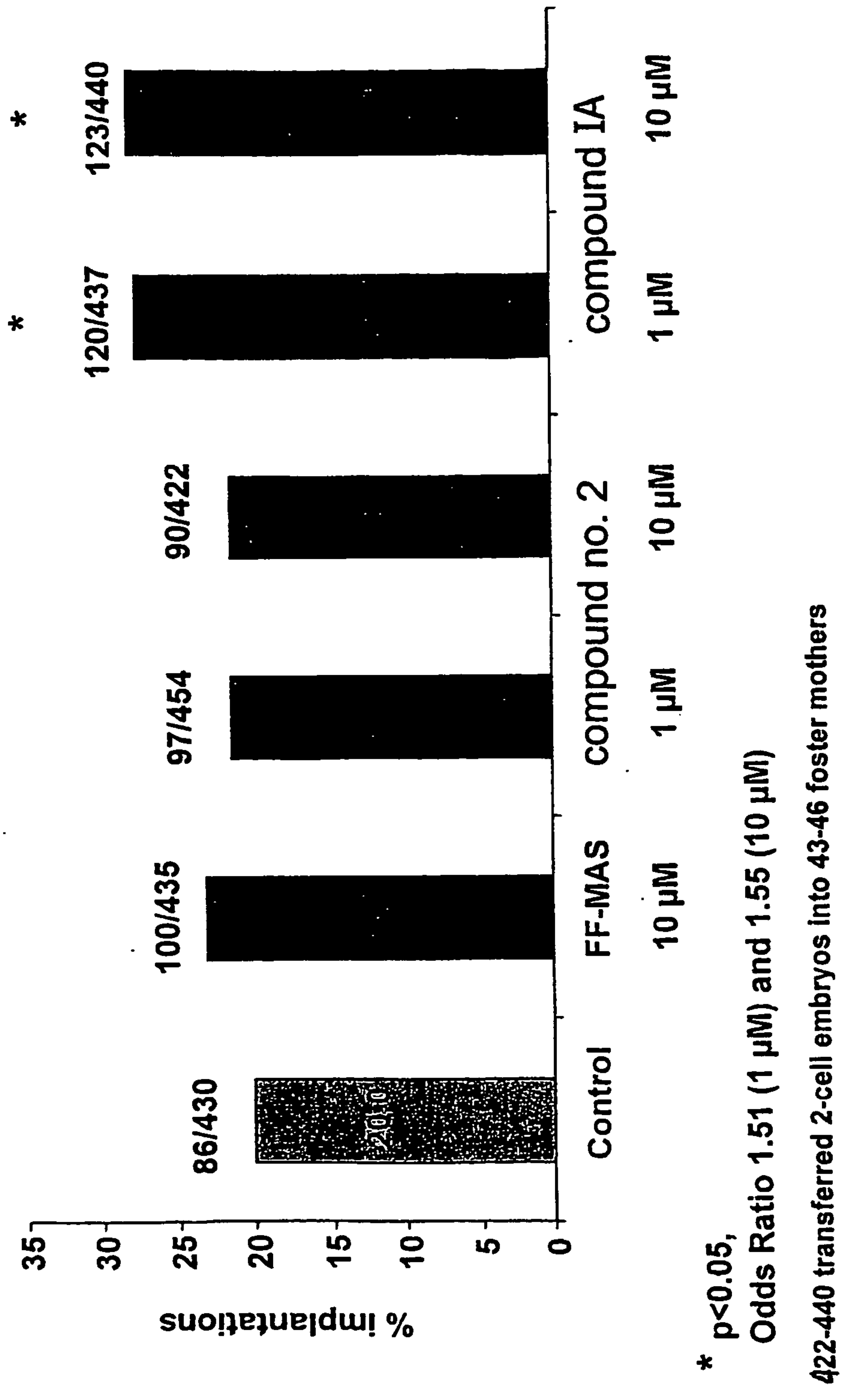
% implantations
0
5
10
15
20
25
30
35
86/430
100/435
97/454
90/422
120/437
123/440
*
*
Control
FF-MAS
10 µM
compound no. 2
1 µM
10 µM
compound IA
1 µM
10 µM
* p<0.05,
Odds Ratio 1.51 (1 µM) and 1.55 (10 µM)
422-440 transferred 2-cell embryos into 43-46 foster mothers

Fig. 4

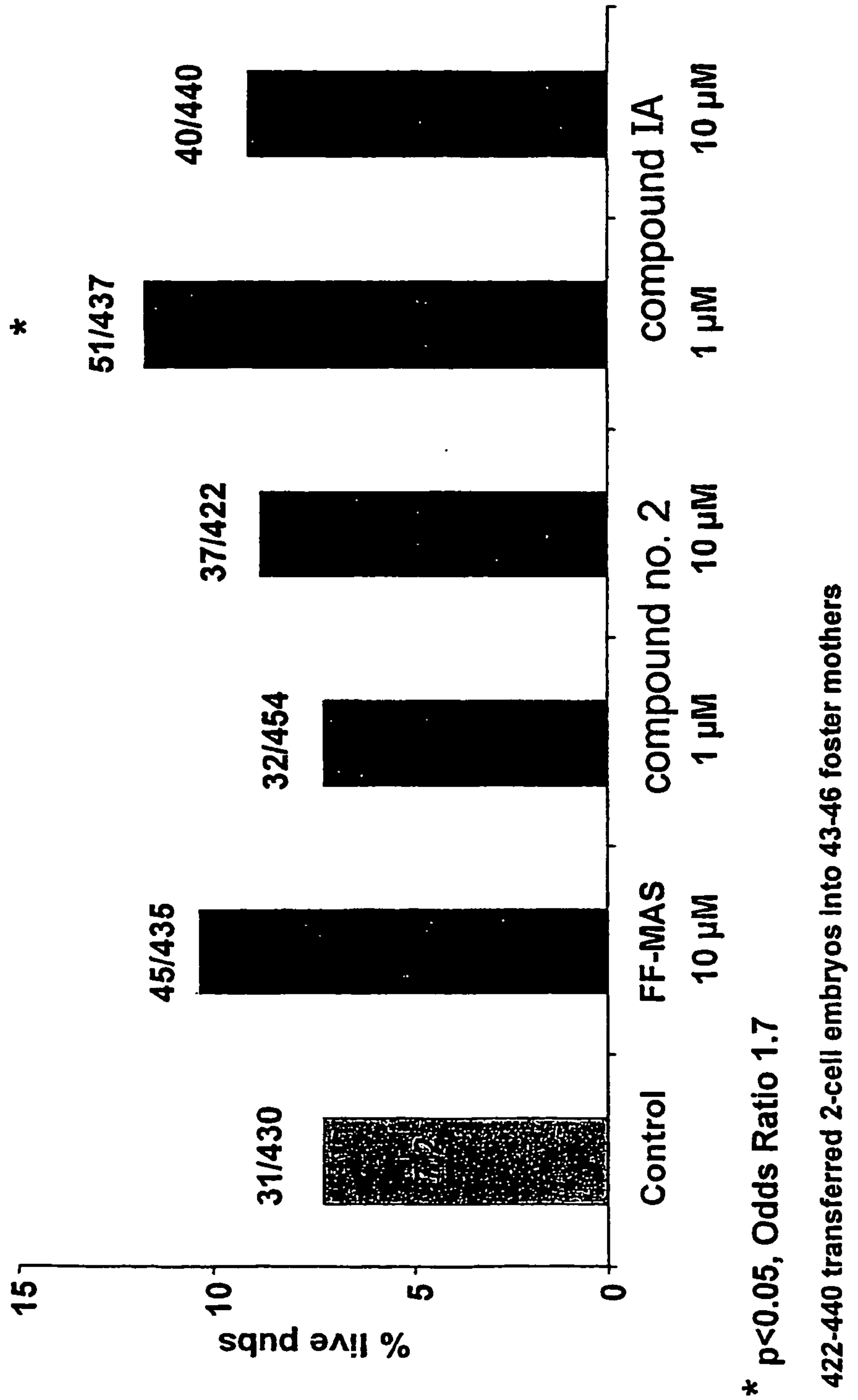
% live pubs
15
10
5
0
31/430
45/435
32/454
37/422
51/437
40/440
*
Control
FF-MAS
10 µM
compound no. 2
1 µM
10 µM
compound IA
1 µM
10 µM
* p<0.05, Odds Ratio 1.7
422-440 transferred 2-cell embryos into 43-46 foster mothers

Fig. 5

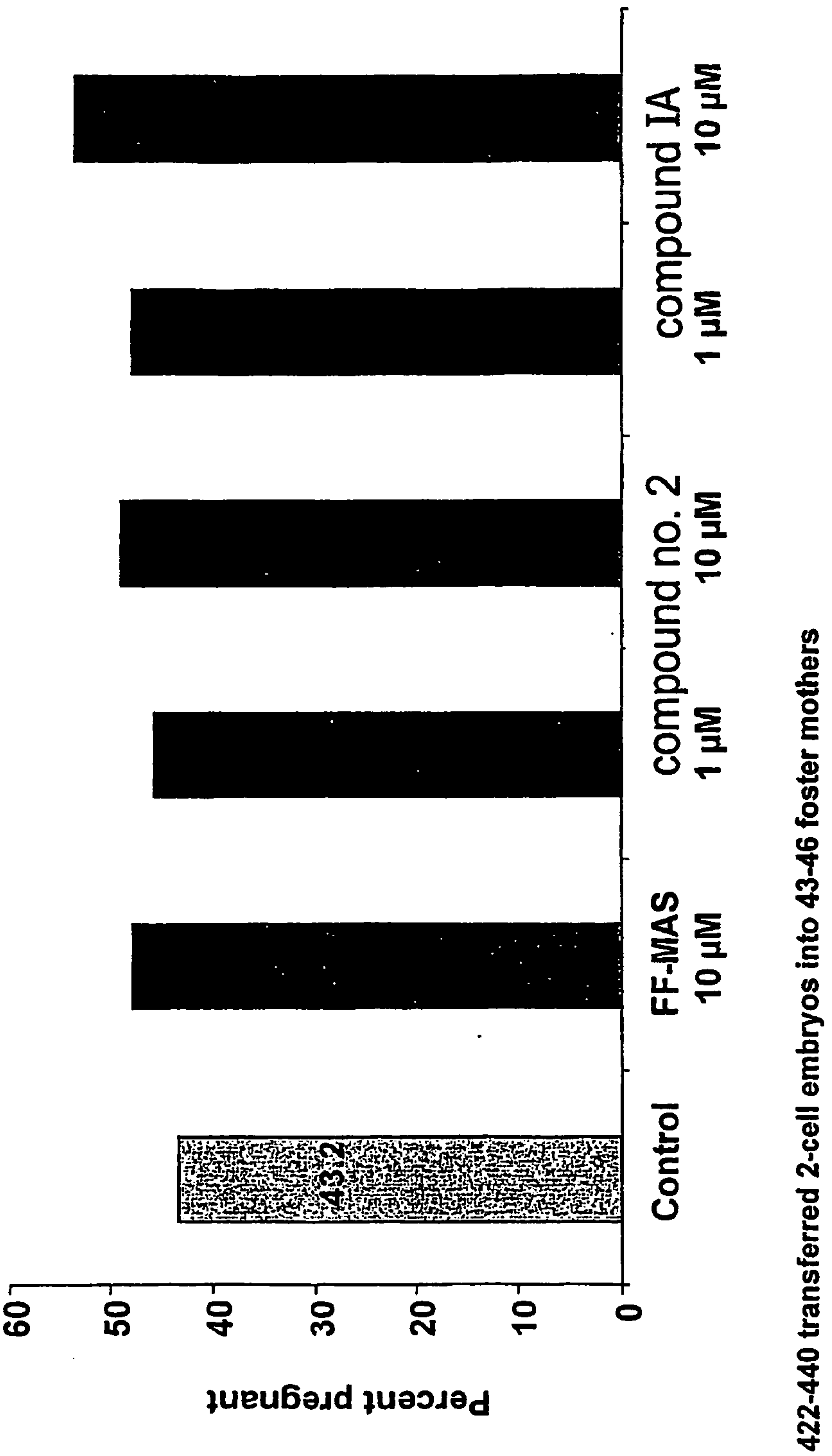
Percent pregnant
0
10
20
30
40
50
60
43.2
Control
FF-MAS
10 µM
compound
1 µM
compound no. 2
10 µM
compound
1 µM
compound IA
10 µM
422-440 transferred 2-cell embryos into 43-46 foster mothers

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9852965 A1
- WO 0068245 A1
- WO 9600235 A1
- WO 9627658 A1
- WO 9700884 A1
- WO 9828323 A1
- WO 9958549 A1
- WO 02079220 A2
- WO 02079200 A2
- WO 9721440 A1


### Non-patent literature cited in the description


- **S.M. DOWNS et al.** *Dev. Biol.,* 1985, vol. 82, 454-458
- **J.J. EPPLG. et al.** *Dev. Biol.,* 1986, vol. 119, 313-321
- **S.M. DOWNS.** *Mol. Reprod. Dev.,* 1993, vol. 35, 82-94
- **A.G. BYSKOV et al.** *Dev. Biol.,* 1976, vol. 52, 193-200
- **A.G. BYSKOV et al.** The Physiology of Reproduction. Raven Press, 1994
- **A.G. BYSKOV et al.** *Nature,* 1995, vol. 374, 559-562
- **C. HEGELE-HARTUNG et al.** *Biol. Reprod.,* 2001, vol. 64, 418-424
- **J.J. SHEETS ; L.E. VICKERY.** Active Site-directed Inhibitors of Cytochrome P-450scc. *J. Biol. Chem.,* 1983, vol. 258 (19), 11446-11452
- **A.T. MANGLA ; W.D. NES.** Sterol C-methyl Transferase from Prototheca wickerhamii, Mechanism, Sterol Specificity and Inhibition. *Bioorg. and Med. Chem.,* 2000, vol. 8 (5), 925-936
- *Journal of Pharmaceutical Science,* 1977, vol. 66, 2
- **L.F. FIESER ; M. FIESER.** Steroids, Reinhold Publishing Corporation. 1959
- Rood's Chemistry of Carbon Compounds. Elsevier Publishing Company, 1971
- Dictionary of Steroids. Chapman & Hall
- *Organic Letters,* 2003, vol. 5, 1837-1839
- **C. GRØNDAHL ; J.L. OTTESEN ; M. LESSI ; P. FAARUP ; A. MURRAY ; FC GRØNVALD ; C. HEGELE-HARTUNG ; I. AHNFELT-RØNNE.** Meiosis-activating sterol promotes resumption of meiosis in mouse oocytes cultured in vitro in contrast to related oxysterols. *Biol. Reprod.,* 1998, vol. 58, 1297-1302
- **C. HEGELE-HARTUNG ; J. KUHNKE ; M. LESSL ; C. GRØNDAHL ; J. OTTESEN ; H.M. BEIER ; S. EISNER ; U. EICHENLAUB-RITTER.** Nuclear and cytoplasmic maturation of mouse oocytes after treatment with synthetic meiosis-activating sterol in vitro. *Biol. Reprod.,* 1999, vol. 61, 1362-1372